# EUROPEAN PATENT APPLICATION

(11) **EP 4 768 592 A1**
(43) Date of publication of application: **01.07.2026**
(21) Application number: 25227549.0
(22) Date of filing: 30.12.2025
(51) Int. Cl.: C12N 15/85

(54) **RAC1 5 UTR-DERIVED NUCLEIC ACID SEQUENCES FOR ENHANCING PROTEIN EXPRESSION**

(30) Priority: 30.12.2024 KR 20240199543
(71) Applicant: Industry-Academic Cooperation Foundation Dankook University, Yongin-si, Gyeonggi-do 16890 (KR)
(72) Inventor: JEONG, Sunjoo, 13531 Seongnam-si, Gyeonggi-do (KR); KIM, Narae, 05078 Seoul (KR); JEONG, Jiwon, 17051 Yongin-si (KR); KIM, Gayoung, 23846 Cheongju-si (KR)
(74) Representative: LLR

(57) **Abstract**

Provided are a 5'UTR sequence for enhancing protein expression and a nucleic acid construct comprising the same. The 5'UTR sequence according to the present disclosure and a nucleic acid construct comprising the same may be employed to effectively enhance the expression of a target protein. This can be accomplished not only through transformation and transfection using DNA plasmids, but also with nucleic acids synthesized through in vitro transcription. The 5'UTR sequence may be utilized in mRNA vaccines and may be applied to the production of therapeutic proteins for specific diseases by incorporating the sequence into various genes to synthesize DNA and RNA constructs.

## Description

### [Technical Field]

The following disclosure relates to a 5' untranslated region (5'UTR) sequence for enhancing protein expression and a nucleic acid construct comprising the same.

### [Background Art]

Messenger RNA (mRNA) serves as a "blueprint" for protein production. mRNA is transcribed from a DNA molecule and essentially contains a coding region (CR) that encodes the protein to be synthesized. mRNA may also contain an untranslated region (UTR). UTRs include a 5'UTR located upstream of the start codon and a 3'UTR located downstream of the stop codon of an mRNA, and refer to sequences that are not translated into a protein. General eukaryotic mRNAs have UTRs at the 5' and 3' ends. UTRs regulate mRNA degradation or translation, exerting substantial effects on mRNA stability and expression, and ultimately playing a key role in controlling protein levels.

The present inventors conducted a study on the protein expression efficiency of the 5'UTR of RAC1, an area that has remained largely unexamined. The present inventors performed fragmentation of the 5'UTR of RAC1 and identified a factor that markedly influenced expression efficiency. Moreover, a sequence that enhances protein expression when combined with the 3'UTR was identified. These findings by the present inventors aim to propose a nucleic acid expression platform system with enhanced protein expression efficiency.

### [Disclosure]

### [Technical Problem]

An embodiment of the present disclosure is directed to providing a nucleic acid construct with enhanced protein translation efficiency.

Another embodiment of the present disclosure is directed to providing a recombinant vector comprising the nucleic acid construct.

Still another embodiment of the present disclosure is directed to providing an isolated host cell comprising the expression vector.

Still another embodiment of the present disclosure is directed to providing a method for producing a nucleic acid with enhanced protein expression efficiency.

### [Technical Solution]

To prevent redundancy and confusion, overlapping descriptions will be omitted below. In other words, the present disclosure is not limited to the embodiments described below and should be interpreted in accordance with the overall scope of the invention.

Hereinafter, the present disclosure will be described in detail.

The present disclosure provides a nucleic acid construct with enhanced protein translation efficiency.

The nucleic acid construct comprises:
(a) a coding region encoding a target protein;
(b) a 5' untranslated region (5'UTR) of Ras-related C3 botulinum toxin substrate 1 (RAC1) or a fragment thereof, linked to the 5' end of the coding region; and
(c) a 3' untranslated region (3'UTR) fragment linked to the 3' end of the coding region.

More specifically, the present disclosure provides a nucleic acid construct comprising (a) a coding region encoding a target protein; (b) a 5'UTR of RAC1 or a fragment thereof, linked to the 5' end of the coding region; and (c) a 3'UTR or a fragment thereof, linked to the 3' end of the coding region.

As used herein, the term "target protein" refers to any protein that is intended to be mass-produced by enhancing its expression efficiency using the nucleic acid construct of the present disclosure. For example, the target protein may be a hormone, a hormone analog, an enzyme, an enzyme inhibitor, a receptor and a receptor fragment, an antigen and an antigen fragment or analog, an antibody and an antibody fragment, a single antibody, a structural protein, a toxin protein, or a protein in which part or all of these are fused.

The term "coding region encoding a target protein" as used herein is a portion of a nucleic acid composed of codons. Codons are translated into proteins according to information provided by the "genetic code." The coding region typically begins with a start codon and ends with a stop codon. Typically, the start codon is an AUG triplet, and the stop codon is UAA, UAG, or UGA. In addition to protein-coding, portions of the coding region may serve as regulatory sequences in pre-mRNA, such as exonic splicing enhancers or exonic splicing silencers.

The coding region encoding the target protein of the present disclosure may include sequences transcribed from various nucleic acid sequences of foreign genes, and may also comprise mutations in nucleotides that do not result in changes in the protein, and thus even if it is a nucleic acid sequence intended to obtain the same target protein, it may include coding regions with various nucleotide mutations. The mutations in nucleotides that do not result in changes in the protein include the nucleic acid molecules that include functionally equivalent codons or codons encoding the same amino acid (e.g., six codons encoding arginine or serine due to codon degeneracy), or codons encoding biologically equivalent amino acids. In consideration of the mutations that retain biologically equivalent activity as described above, the nucleic acid molecules used in the present disclosure are interpreted to also encompass sequences that exhibit substantial identity with the sequences listed in the Sequence Listing. By using a nucleic acid construct in which the 5'UTR and 3'UTR of the present disclosure or a fragment thereof is linked to the coding region of an mRNA sequence capable of translating a protein to be introduced into a subject, the translation level of the protein may be significantly enhanced.

The nucleic acid construct according to the present disclosure may be in RNA or DNA form. Preferably, the nucleic acid construct according to the present disclosure is in RNA form, which may provide superior efficacy compared to DNA forms. It is particularly advantageous for use in view of in vitro transcription (IVT) mRNA expression systems.

First, unlike nucleic acid molecules in DNA form, RNA molecules do not require entry into the host cell nucleus for transcription, as they can be directly translated in the cytoplasm. Accordingly, RNA molecules enable cytoplasmic synthesis of peptides or proteins without nuclear localization. Therefore, nucleic acid molecules in RNA form may be more suitable than nucleic acid molecules in DNA form from the perspective of nucleic acid delivery and expression. Specifically, RNA molecules have a minimal likelihood of integrating into the host chromosome. In addition, antibiotic resistance genes, which are selectable markers used for selective production in host cells, are unnecessary for RNA nucleic acid production. Furthermore, the intrinsically shorter half-life of RNA compared with DNA does not induce long-term or persistent genetic modification. In other words, nucleic acid molecules in their general form are delivered into cells, expressed for a short period of time to express target peptides/proteins, and are degraded by enzymatic reactions within a few days, but a specific immune response elicited by the initially expressed targets peptides/proteins persists.

Second, nucleic acid molecules in RNA form may induce a desired immune response in vivo even at relatively lower amounts compared to DNA. As mentioned above, unlike DNA, RNA molecules do not need to enter the nucleus and only need to pass through the cell membrane. Consequently, RNA molecules are capable of achieving comparable expression levels of target peptides or proteins as DNA molecules, even at lower amounts.

Third, the ability to artificially control all manufacturing processes allows for safe vaccine production in small-scale GMP (Good Manufacturing Practice) facilities without the risk of biological contamination. In other words, nucleic acid molecules in RNA form require only small-scale GMP laboratory-level facilities and do not require direct handling of infectious agents (viruses or pathogenic microorganisms), enabling rapid manufacturing and production of a variety of vaccines.

Fourth, nucleic acid molecules in RNA form may induce a stronger immune response than naked DNA nucleic acid molecules. This is thought to result from the cooperative interaction between the innate immune response induced by the stem-loop structure of RNA molecules and the inherent properties of RNA itself, and the adaptive immune response induced by target peptides/proteins expressed in the immunized host cell.

Fifth, RNA molecules are easy to produce. As described above, large quantities of RNA molecules may be produced solely through in vitro transcription (IVT). Recent improvements in IVT reagents, particularly DNA-dependent RNA polymerases, have enabled rapid production of large quantities of RNA within one to two weeks using small amounts of DNA template. Further, multiple antigens intended to induce an immune response may be simultaneously produced, mixed, and then used for immunization.

Under these circumstances, the nucleic acid construct according to the present disclosure is also highly useful in the context of an IVT mRNA expression system.

The "UTR (untranslated region)" of the present disclosure refers to an untranslated sequence located upstream of the start codon and downstream of the stop codon of an mRNA. The UTR located upstream of the start codon of an mRNA is referred to as the 5'UTR, and the UTR located downstream of the stop codon of an mRNA is referred to as the 3'UTR. The untranslated region of mRNA plays a pivotal role in regulating both mRNA stability and mRNA translation.

As used herein, "5'UTR of RAC1" refers to the 5'UTR of the mRNA of the RAC1 gene.

As used herein, "3'UTR of β-catenin" refers to the 3'UTR of the mRNA of the CTNNB1 (β-catenin) gene.

As used herein, "pUC vector-derived 3'UTR" refers to the 3'UTR derived from a pUC vector (pUC 3'UTR). In the present specification, it may be used interchangeably with "pUC 3'UTR".

As used herein, "3'UTR of β-actin" refers to the 3' untranslated region of the mRNA for the ACTB (β-actin) gene.

As used herein, "3'UTR of human β-globin (HBB)" refers to the 3' untranslated region of the mRNA for the human β-globin gene.

"Ras-related C3 botulinum toxin substrate 1 (RAC1)" as used herein refers to a small (~21 kDa) GTPase protein belonging to the Rac subfamily of the Rho family, which regulates various cellular processes, including cytoskeletal formation, cell migration, and cell proliferation. Furthermore, RAC1 plays a central role in intracellular signaling networks and is known to interact with other signaling molecules to regulate cell behavior.

As used herein, the "5'UTR of RAC1 or a fragment thereof" refers to a sequence validated by the inventors of the present disclosure for translation efficiency to optimize the mRNA 5'UTR sequence of the RAC1 gene, including, for example, fragments possessing stable stem-loop structures identified through RNA structural analysis. Here, the term "stem-loop structure" refers to a structure composed of a double-stranded RNA structure region (stem) formed by hydrogen bonds between inverted repeat sequences existing on a single-stranded RNA and a single-stranded RNA loop portion between them where no hydrogen bonds are formed, which is also commonly called a hairpin loop structure.

Specifically, in the present disclosure, the 5'UTR of RAC1 or a fragment thereof may comprise any one sequence selected from the group consisting of SEQ ID NOs: 30 to 36.

Further, the 5'UTR of RAC1 may comprise mutants derived from the wild-type (WT) RAC1 5'UTR. The mutants may be those in which one or more guanine (G) residues within a G-rich sequence capable of forming a G-quadruplex (GQ) structure are substituted with adenine (A), uracil (U), or cytosine (C).

According to an embodiment, the RAC1 5'UTR mutant sequence of the present disclosure may comprise one, two, or three guanines (G) located within the G-rich region with a high G-score, after predicting a G-quadruplex structure, substituted with adenine (A), uracil (U), or cytosine (C).

According to an embodiment of the present disclosure, the RAC1 5'UTR mutant sequence may comprise substitution with adenine (A).

The use of such mutant sequences may significantly increase translation efficiency.

According to an embodiment of the present disclosure, the sequence in which three guanines (G) are substituted with adenine (A) exhibited the highest translation efficiency among the mutant sequences.

In the present disclosure, the 5'UTR of RAC1 or a fragment thereof may have any one sequence selected from the group consisting of SEQ ID NOs: 30 to 36 below.

In the present disclosure, the 5'UTR of RAC1 or a fragment thereof may consist essentially of any one sequence selected from the group consisting of SEQ ID NOs: 30 to 36 below.

In the present disclosure, the 5'UTR of RAC1 or a fragment thereof may consist of any one sequence selected from the group consisting of SEQ ID NOs: 30 to 36 below.
5'UTR region of RAC1 (FL): 
CTGTTTCTCTGCAGTTTTCCTCAGCTTTGGGTGGTGGCCGCTGCCGGGCATCGGCTTCC

AGTCCGCGGAGGGCGAGGCGGCGTGGACAGCGGCCCCGGCACCCAGCGCCCCGCCGCCCGCAAG

CCGCGCGCCCGTCCGCCGCGCCCCGAGCCCGCCGCTTCCTATCTCAGCGCCCTGCCGCCGCCGC
 CGCGGCCCAGCGAGCGGCCCTG (SEQ ID NO: 30)
A fragment of RAC1 5'UTR (SL1):
   AGTTTTCCTCAGCTTTGGGTGGTGGCCGCTGCCGGGCATCGGCTTCCAGTCCGC (SEQ ID NO: 31)
A fragment of RAC1 5'UTR (SL2): 
GGAGGGCGAGGCGGCGTGGACAGCGGCCCCGGCACCCAGCGCCCCGCCGCCCGCAAGCC

GCGCGCCCGTCC (SEQ ID NO: 32)
A fragment of RAC1 5'UTR (SL3): 
GCCGCGCCCCGAGCCCGCCGCTTCCTATCTCAGCGCCCTGCCGCCGCCGCGGCCCAGCGAG

CGGCCCTG (SEQ ID NO: 33)
A fragment of RAC1 5'UTR (SL1+2): 
AGTTTTCCTCAGCTTTGGGTGGTGGCCGCTGCCGGGCATCGGCTTCCAGTCCGCGGAGGGCGAG

GCGGCGTGGACAGCGGCCCCGGCACCCAGCGCCCCGCCGCCCGCAAGCCGCGCGCCCGTCC

(SEQ ID NO: 34)
A fragment of RAC1 5'UTR (SL2+3): 
GGAGGGCGAGGCGGCGTGGACAGCGGCCCGGCACCCAGCGCCCGCCGCCCGCAAGCCGCGCG

CCCGTCCGCCGCGCCCCGAGCCCGCCGCTTCCTATCTCAGCGCCCTGCCGCCGCCGCGGCC

CAGCGAGCGGCCCTG (SEQ ID NO: 35)
A fragment of RAC1 5'UTR (SL1+3):

AGTTTTCCTCAGCTTTGGGTGGTGGCCGCTGCCGGGCATCGGCTTCCAGTCCGCGCCGCGCCCC

GAGCCCGCCGCTTCCTATCTCAGCGCCCTGCCGCCGCCGCGGCCCAGCGAGCGGCCCTG

(SEQ ID NO: 36)

In the present disclosure, the 5'UTR mutant of RAC1 is a mutant of the full-length sequence of the 5'UTR region of RAC1, and may comprise any one sequence selected from the group consisting of SEQ ID NOs: 52 to 54 below.
Mutant of the 5'UTR of RAC1 (GA1): 
AGTTTTCCTCAGCTTTGGGTGGTGGCCGCTGCCGGGCATCGGCTTCCAGTCCGCGGAGG

GCGAGGCAGCGTGGACAGCGGCCCCGGCACCCAGCGCCCCGCCGCCCGCAAGCCGCGCGCCCGT

CCGCCGCGCCCCGAGCCCGCCGCTTCCTATCTCAGCGCCCTGCCGCCGCCGCGGCCCAGCG

AGCGGCCCTG (SEQ ID NO: 52)
Mutant of the 5'UTR of RAC1 (GA2): 
AGTTTTCCTCAGCTTTGGGTGGTGGCCGCTGCCGGGCATCGGCTTCCAGTCCGCGGAGG

GCGAGGCAGCGTAGACAGCGGCCCCGGCACCCAGCGCCCCGCCGCCCGCAAGCCGCGCGCCCGT

CCGCCGCGCCCCGAGCCCGCCGCTTCCTATCTCAGCGCCCTGCCGCCGCCGCGGCCCAGCG

AGCGGCCCTG (SEQ ID NO: 53)
Mutant of the 5'UTR of RAC1 (GA3): 
AGTTTTCCTCAGCTTTGGGTGGTGGCCGCTGCCGGGCATCGGCTTCCAGTCCGCGGAGG

GCGAGGCAGCGTAGACAGCAGCCCCGGCACCCAGCGCCCCGCCGCCCGCAAGCCGCGCGCCCGT

CCGCCGCGCCCCGAGCCCGCCGCTTCCTATCTCAGCGCCCTGCCGCCGCCGCGGCCCAGCG

AGCGGCCCTG (SEQ ID NO: 54)
   ** Bold indicates substitution sites.*

Further, in the present disclosure, the 5'UTR mutant of RAC1 is a mutant of a fragment of the 5'UTR region of RAC1, and may comprise any one sequence selected from the group consisting of SEQ ID NO: 61 to 69 below.
Mutant of the 5'UTR fragment (SL2) of RAC1 (SL2_GA1): 
GGAGGGCGAGGCAGCGTGGACAGCGGCCCCGGCACCCAGCGCCCCGCCGCCCGCAAGCC

GCGCGCCCGTCC (SEQ ID NO: 61)
Mutant of the 5'UTR fragment (SL2) of RAC1 (SL2_GA2): 
GGAGGGCGAGGCAGCGTAGACAGCGGCCCCGGCACCCAGCGCCCCGCCGCCCGCAAGCC

GCGCGCCCGTCC (SEQ ID NO: 62)
Mutant of the 5'UTR fragment (SL2) of RAC1 (SL2_GA3): GGAGGGCGAGGCAGCGTAGACAGCAGCCCCGGCACCCAGCGCCCCGCCGCCCGCAAGCC GCGCGCCCGTCC (SEQ ID NO: 63)
Mutant of the 5'UTR fragment (SL1+2) of RAC1 (SL1+2_GA1): 
AGTTTTCCTCAGCTTTGGGTGGTGGCCGCTGCCGGGCATCGGCTTCCAGTCCGCGGAGG
GCGAGGCAGCGTGGACAGCGGCCCCGGCACCCAGCGCCCCGCCGCCCGCAAGCCGCGCGCCCGT
CC (SEQ ID NO: 64)
Mutant of the 5'UTR fragment (SL1+2) of RAC1 (SL1+2_GA2): 
AGTTTTCCTCAGCTTTGGGTGGTGGCCGCTGCCGGGCATCGGCTTCCAGTCCGCGGAGG

GCGAGGCAGCGTAGACAGCGGCCCCGGCACCCAGCGCCCCGCCGCCCGCAAGCCGCGCGCCCGT

CC (SEQ ID NO: 65)
Mutant of the 5'UTR fragment (SL1+2) of RAC1 (SL1+2_GA3): 
AGTTTTCCTCAGCTTTGGGTGGTGGCCGCTGCCGGGCATCGGCTTCCAGTCCGCGGAGG

GCGAGGCAGCGTAGACAGCAGCCCCGGCACCCAGCGCCCCGCCGCCCGCAAGCCGCGCCCGT

CC (SEQ ID NO: 66)
Mutant of the 5'UTR fragment (SL2+3) of RAC1 (SL2+3_GA1): 
GGAGGGCGAGGCAGCGTGGACAGCGGCCCCGGCACCCAGCGCCCCGCCGCCCGCAAGCC

GCGCGCCCGTCCGCCGCGCCCCGAGCCCGCCGCTTCCTATCTCAGCGCCCTGCCGCCGCCG

CGGCCCAGCGAGCGGCCCTG (SEQ ID NO: 67)
Mutant of the 5'UTR fragment (SL2+3) of RAC1 (SL2+3_GA2): 
GGAGGGCGAGGCAGCGTAGACAGCGGCCCCGGCACCCAGCGCCCCGCCGCCCGCAAGCC

GCGCGCCCGTCCGCCGCGCCCCGAGCCCGCCGCTTCCTATCTCAGCGCCCTGCCGCCGCCG

CGGCCCAGCGAGCGGCCCTG (SEQ ID NO: 68)
Mutant of the 5'UTR fragment (SL2+3) of RAC1 (SL2+3_GA3): 
GGAGGGCGAGGCAGCGTAGACAGCAGCCCCGGCACCCAGCGCCCCGCCGCCCGCAAGCC

GCGCGCCCGTCCGCCGCGCCCCGAGCCCGCCGCTTCCTATCTCAGCGCCCTGCCGCCGCCG

CGGCCCAGCGAGCGGCCCTG (SEQ ID NO: 69)
   ** Bold indicates substitution sites.*

In the present disclosure, the 3'UTR or a fragment thereof may be selected from, but is not limited to, for example, β-catenin, a pUC vector-derived 3'UTR, β-actin, human β-globin (HBB), albumin, human α-globin (HBA), alanine aminotransferase 1, human apolipoprotein E, human antithrombin, etc.

According to an embodiment of the present disclosure, the 3'UTR or a fragment thereof may be any one 3'UTR or fragment thereof, selected from the group consisting of β-catenin, a pUC vector-derived 3'UTR, β-actin, and human β-globin.

According to an embodiment, the RAC1 5'UTR is not limited to a specific 3'UTR and may provide a stable and consistent translation enhancing effect even when combined with the 3'UTRs of various genes (e.g., β-catenin, pUC vector-derived sequence, β-actin, or human β-globin)

As used herein, "β-catenin," also known as Catenin beta-1, refers to a protein expressed from the human CTNNB1 gene. β-catenin is a subunit of the cadherin protein complex and is known as a key factor in Wnt cell signaling, which is involved in cell fate determination, cell migration, and regulation of cell polarity. The human CTNNB1 gene is accessible through known databases such as GenBank (CTNNB1, catenin beta 1 [Homo sapiens (human)], Gene ID: 1499).

In the present disclosure, the term "β-catenin 3'UTR (or expressed as 3'UTR of β-catenin) or a fragment thereof" refers to a sequence validated by the inventors of the present disclosure for translation efficiency to optimize the 3'UTR sequence of the mRNA of the β-catenin gene, and may include a mutant resulting from alternative splicing of the 3'UTR. In this specification, the terms "β-catenin 3'UTR fragment," "β-catenin-derived 3'UTR fragment," "CTNNB1 3'UTR fragment," and "CTNNB1-derived 3'UTR fragment" are used interchangeably.

Specifically, in the present disclosure, the β-catenin 3'UTR or a fragment thereof may comprise any one sequence selected from the group consisting of SEQ ID NOs: 23 to 25.

In the present disclosure, the β-catenin 3'UTR or a fragment thereof may have any one sequence selected from the group consisting of SEQ ID NOs: 23 to 25.

In the present disclosure, the β-catenin 3'UTR or a fragment thereof may consist essentially of any one sequence selected from the group consisting of SEQ ID NOs: 23 to 25.

In the present disclosure, the β-catenin 3'UTR or a fragment thereof may consist of any one sequence selected from the group consisting of SEQ ID NOs: 23 to 25.
β-catenin 3'UTR or a fragment thereof (F1): 
CTGTATTGTCTGAACTTGCATTGTGATTGGCCTGTAGAGTTGCTGAGAGGGCTCGAGGGGTGGG

CTGGTATCTCAGAAAGTGCCTGACACACTAACCAAGCTGAGTTTCCTATGGGAACAATTGAAGT
 
AAACTTTTTGTTCTGGTCCTTTTTGGTCGAGGAGTAACAATACAAATGGATTTTGGGAGTGACT

CAAGAAGTGAAGAATGCACAAGAATGGATCACAAGATGGAATTTATCAAACCCTAGCCTTGCTT

GTTAAATTTTTTTTTTTTTTTTTTTAAGAATATCTGTAATGGTACTGACTTTGCTTGCTTTGAA

GTAGCTCTTTTTTTTTTTTTTTTTTTTTTTTTGCAGTAACTGTTTTTTAAGTCTCTC (SEQ ID

NO: 23)
β-catenin 3'UTR or a fragment thereof (F3): 
GTAACTGTTTTTTAAGTCTCTCGTAGTGTTAAGTTATAGTGAATACTGCTACAGCAATTTCTAA

TTTTTAAGAATTGAGTAATGGTGTAGAACA/CTAATTCATAATCACTCTAATTAATTGTAATCT

GAATAAAGTGTAACAATTGTGTAGCCTTTTTGTATAAAATAGACAAATAGAAAATGGTCCAATT

AGTTTCCTTTTTAATATGCTTAAAATAAGCAGGTGGATCTATTTCATGTTTTTGATCAAAAACT

ATTTGGGATATGTATGGGTAGGGTAAATCAGTAAGAGGTGT (SEQ ID NO: 24)
β-catenin 3'UTR or a fragment thereof (F1+3):

CTGTATTGTCTGAACTTGCATTGTGATTGGCCTGTAGAGTTGCTGAGAGGGCTCGAGGGGTGGG

CTGGTATCTCAGAAAGTGCCTGACACACTAACCAAGCTGAGTTTCCTATGGGAACAATTGAAGT

AAACTTTTTGTTCTGGTCCTTTTTGGTCGAGGAGTAACAATACAAATGGATTTTGGGAGTGACT

CAAGAAGTGAAGAATGCACAAGAATGGATCACAAGATGGAATTTATCAAACCCTAGCCTTGCTT

GTTAAATTTTTTTTTTTTTTTTTTTAAGAATATCTGTAATGGTACTGACTTTGCTTGCTTTGAA

GTAGCTCTTTTTTTTTTTTTTTTTTTTTTTTTGCAGTAACTGTTTTTTAAGTCTCTCGTAACTG

TTTTTTAAGTCTCTCGTAGTGTTAAGTTATAGTGAATACTGCTACAGCAATTTCTAATTTTTAA

GAATTGAGTAATGGTGTAGAACA/CTAATTCATAATCACTCTAATTAATTGTAATCTGAATAAA

GTGTAACAATTGTGTAGCCTTTTTGTATAAAATAGACAAATAGAAAATGGTCCAATTAGTTTCC

TTTTTAATATGCTTAAAATAAGCAGGTGGATCTATTTCATGTTTTTGATCAAAAACTATTTGGG

ATATGTATGGGTAGGGTAAATCAGTAAGAGGTGT (SEQ ID NO: 25)

The 3'UTR may be selected from, for example, β-actin, a pUC vector-derived 3'UTR, or human β-globin, but is not limited thereto. These sequences are represented by SEQ ID NO: 43, 44, and 45, respectively, below.
3'UTR of β-actin: 
GCGGACTATGACTTAGTTGCGTTACACCCTTTCTTGACAAAACCTAACTTGCGCAGAAA

ACAAGATGAGATTGGCATGGCTTTATTTGTTTTTTTTGTTTTGTTTTGGTTTTTTTTTTTTTTT

TGGCTTGACTCAGGATTTAAAAACTGGAACGGTGAAGGTGACAGCAGTCGGTTGGAGCGAGCAT

CCCCCAAAGTTCACAATGTGGCCGAGGACTTTGATTGCACATTGTTGTTTTTTTAATAGTCATT

CCAAATATGAGATGCGTTGTTACAGGAAGTCCCTTGCCATCCTAAAAGCCACCCCACTTCTCTC

TAAGGAGAATGGCCCAGTCCTCTCCCAAGTCCACACAGGGGAGGTGATAGCATTGCTTTCGTGT

AAATTATGTAATGCAAAATTTTTTTAATCTTCGCCTTAATACTTTTTTATTTTGTTTTATTTTG

AATGATGAGCCTTCGTGCCCCCCCTTCCCCCTTTTTTGTCCCCCAACTTGAGATGTATGAAGGC
 
TTTTGGTCTCCCTGGGAGTGGGTGGAGGCAGCCAGGGCTTACCTGTACACTGACTTGAGACCAG

TTGAATAAAAGTGCACACCTTAAAAATGA (SEQ ID NO: 43)
3'UTR sequence derived from pUC vector: 
AGGATACAGCGGCTTCTGCGCGACTTATAAGAGCTCCTTGTGCGGCGCCATTTTAAGCC

TCTCGGTCTGTGGCAGCAGCGTTGGCCCGGCCCCGGGAGCGGAGAGCGAGGGGAGGCGGAGACG

GAGGAAGGTCTGAGGAGCAGCTTCAGTCCCCGCCGAGCCGCCACCGCAGGTCGAGGACGGTCGG

ACTCCCGCGGCGGGAGGAGCCTGTTCCCCTGAGGGTATTTGAAGTATACCATACAACTGTTTTG

AAAATCCAGCGTGGACA (SEQ ID NO: 44)
3'UTR of human β-globin (HBB):

GCTCGCTTTCTTGCTGTCCAATTTCTATTAAAGGTTCCTTTGTTCCCTAAGTCCAACTA
CTAAACTGGGGGATATTATGAAGGGCCTTGAGCATCTGGATTCTGCCTAATAAAAAACATTTAT
TTTCATTGC (SEQ ID NO: 45).

Depending on the type of the 5'UTR, 3'UTR, or fragment thereof, the location and translation efficiency of the nucleic acid construct may vary.

According to an embodiment, for DNA, translation efficiency is significantly enhanced when either (i) a combination of the 5'UTR region of RAC1 (FL) and the fragment of β-catenin 3'UTR (F3 or F1+3) or (ii) a combination of the fragment of RAC1 5'UTR (SL3) and the fragment of β-catenin 3'UTR (F3) is employed.

In addition, translation efficiency is significantly enhanced when GA3 is used as a mutant of the 5'UTR of RAC1. In particular, translation may be significantly enhanced when either (i) a combination of a mutant of the 5'UTR of RAC1 (GA3) and a fragment of β-catenin 3'UTR (F3), or (ii) a combination of a mutant of the 5'UTR of RAC1 (GA3) and a fragment of β-catenin 3'UTR (F1+3) is employed.

According to an embodiment, for RNA, translation efficiency is significantly enhanced when either (i) FL, the 5'UTR region of RAC1, is used with F1 or F3 as the fragment of β-catenin 3'UTR, or (ii) SL3, the fragment of RAC1 5'UTR, is used with F1 or F3 as the fragment of β-catenin 3'UTR.

In the present disclosure, the 5'UTR or a fragment thereof may comprise any one sequence selected from the group consisting of SEQ ID NOs: 30 to 36, and the 3'UTR of β-catenin or a fragment thereof may comprise any one sequence selected from the group consisting of SEQ ID NOs: 23 to 25. More specifically, the 5'UTR or a fragment thereof may comprise, have, consist essentially of, or consist of the above-described sequences.

The 5'UTR, 3'UTR, and fragments thereof used in the present disclosure are also interpreted to include sequences that exhibit substantial identity with the sequences set forth in SEQ ID NOs: 30 to 36 and SEQ ID NOs: 23 to 25, respectively. The above substantial identity refers to a sequence that exhibits at least 60% homology (e.g., 60%, 61%, 62%, 63%, 64%, 65%, 66%, 67%, 68%, or 69%), more preferably 70% homology (e.g., 70%, 71%, 72%, 73%, 74%, 75%, 76%, 77%, 78%, or 79%), even more preferably 80% homology (e.g., 80%, 81%, 82%, 83%, 84%, 85%, 86%, 87%, 88%, or 89%), and most preferably 90% homology (e.g., 90%, 91%, 92%, 93%, 94%, 95%, 96%, 97%, 98%, 99%, or 100%) when the sequence of the present disclosure and any other sequence are aligned as closely as possible and the aligned sequence is analyzed using an algorithm commonly used in the art. All integers greater than or equal to 70% and less than or equal to 100%, as well as decimals therebetween, are included within the scope of the present disclosure with respect to percent homology.

Alignment methods for sequence comparison are well known in the art. Various alignment methods and algorithms are described in the document [Smith and Waterman, Adv. Appl. Math. 2:482(1981) Needleman and Wunsch, J. Mol. Bio. 48:443(1970); Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992) and Pearson et al., Meth. Mol. Biol. 24:307-31(1994)]. The NCBI Basic Local Alignment Search Tool (BLAST) (Altschul et al., J. Mol. Biol. 215:403-10 (1990)) is available from the National Center for Biotechnology Information (NCBI) and can be used online in conjunction with sequence analysis programs such as blastp, blastn, blastx, tblastn, and tblastx. BLAST can be accessed through the BLAST page on the NCBI website. Methods for sequence homology comparison using this program can be found on the BLAST Help page of the NCBI website.

The nucleic acid construct of the present disclosure may comprise a 5' cap structure at the 5' end of the 5'UTR, a poly(A) tail structure at the 3' end of the 3'UTR, or both.

More specifically, the nucleic acid construct of the present disclosure may comprise a 5' cap structure at the 5' end of the 5'UTR, and may further comprise a poly(A) tail structure at the 3' end of the 3'UTR.

Specifically, the poly (A) tail refers to a base sequence in which multiple adenosine monophosphates (AMP) are linked via phosphodiester bonds, and may have a length of 20 nt to 500 nt, for example, 30 nt to 450 nt, 50 nt to 400 nt, 100 nt to 350 nt, or 150 nt to 310 nt. It may be directly attached to the 3' end of the coding sequence encoding the target protein, or arbitrary base sequence may be inserted between the 3' end of the coding sequence encoding the target protein and the poly (A) tail. This allows the nucleic acid construct to not only exhibit high structural stability within the cytoplasm, but also actively induce the expression of a fully functional target protein for a prolonged period of time, thereby further enhancing the translation efficiency of the nucleic acid construct.

Furthermore, the nucleic acid construct of the present disclosure may comprise one or more modified natural nucleotides, which are known to improve stability and enhance translation. Such modified nucleotides may include, for example, N1-methylpseudo uridine and 5-methylcytosine. It will be apparent to those skilled in the art that modifications to the nucleic acid constructs of the present disclosure are permissible within the scope described herein. Therefore, the nucleic acid construct according to the present disclosure may include any one or more modified nucleotides selected from the group consisting of N1-methylpseudo uridine, and 5-methylcytosine.

Optionally, the nucleic acid construct according to the present disclosure further comprises a nucleic acid sequence encoding a signal peptide, if desired. Preferably, the signal peptide is in-frame with the nucleic acid sequence encoding the signal peptide and may be arranged between the 5'UTR and the coding region encoding the target protein. A signal peptide is a peptide that directly or indirectly mediates the transport or secretion of a polypeptide or a protein fused or attached to the signal peptide into the extracellular space, and therefore, preferably, the signal sequence is cleaved from the polypeptide and the protein, respectively.

Optionally, nucleic acid sequences capable of increasing the expression efficiency of the coding region (CR) linked in the form of an open reading frame (ORF) may be further inserted. For example, multiple adenosines (MA) or nucleotides capable of being transcribed into multiple adenosines (MA) may be inserted. For example, multiple adenosines (MA) may be inserted at the 5' end of the expression control sequence of the target protein in the form of an RNA transcript, but the insertion sites for the multiple adenosines (MA) are not limited to these positions.

The nucleic acid construct of the present disclosure may be used in various ways, such as for direct injection into a subject or for in vitro cell application. It can be utilized for a variety of purposes, including therapeutics, vaccines, disease diagnosis, and drug screening, all of which utilize nucleic acid sequences. The nucleic acid construct of the present disclosure may be employed by various conventionally known methods to enhance its stability, such as intracellular delivery using a phospholipid membrane-encapsulating approach.

As described above, the nucleic acid construct of the present disclosure, in its RNA form, exhibits superior efficacy compared to its DNA form. It is particularly advantageous for use in an IVT mRNA expression system, and its application should be considered separately from that in conventional vector-based systems.

As used herein, the term "nucleic acid" encompasses both DNA (gDNA and cDNA) and RNA molecules. Nucleotides, the fundamental structural units of nucleic acid molecules, include not only natural nucleotides but also analogs with modifications in sugar or base moieties (Scheit, Nucleotide Analogs, John Wiley, New York(1980); Uhlman and Peyman, Chemical Reviews, 90:543-584(1990)).

The coding region (CR) of the nucleic acid construct according to the present disclosure may be a sequence transcribed from a foreign nucleic acid sequence, and may comprise mutations in nucleotides that do not result in changes in the protein, and thus even if it is a nucleic acid sequence intended to obtain the same target protein, it may include coding region (CR) with various nucleotide mutations. The mutations in nucleotides that do not result in changes in the protein include the nucleic acid molecules that include functionally equivalent codons or codons encoding the same amino acid (e.g., six codons encoding arginine or serine due to codon degeneracy), or codons encoding biologically equivalent amino acids. In consideration of the mutations that retain biologically equivalent activity as described above, the nucleic acid molecules used in the present disclosure are interpreted to also encompass sequences that exhibit substantial identity with the sequences listed in the Sequence Listing. For the 5'UTR and 3'UTR or fragments thereof, these regions correspond to untranslated regions and relatively limited modifications may be permitted compared to the coding region (CR); however, minor modifications that do not substantially affect translation activity are considered acceptable by those skilled in the art.

Nucleic acid constructs according to the present disclosure may include various conventionally known promoters. The promoter is operatively linked to the nucleic acid sequence of the present disclosure to control the transcription and/or translation of the nucleic acid sequence.

The term "operatively linked" refers to a functional linkage between a nucleic acid expression control sequence, such as a promoter, signal sequence, or an array of transcription factor binding sites, and another nucleic acid sequence, whereby the control sequence regulates the transcription and/or translation of the other nucleic acid sequence.

The present disclosure provides a recombinant vector comprising the nucleic acid construct.

The vector system of the present disclosure may be constructed using various methods known in the art. Specific methods thereof are disclosed in Sambrook, et al., Cold Spring Harbor Laboratory Press (2001), which is incorporated herein by reference.

The vector of the present disclosure may typically be constructed as a cloning vector or an expression vector. Furthermore, the vector of the present disclosure may be constructed using either prokaryotic or eukaryotic cells as the host. For example, when the vector of the present disclosure is an expression vector and a prokaryotic cell is used as the host, it typically comprises a strong promoter capable of driving transcription (e.g., the pLλ promoter, the trp promoter, the lac promoter, the T7 promoter, the tac promoter, etc.), a ribosome binding site for translation initiation, and a transcription/translation termination sequence. If *E. coli* is used as the host cell, the promoter and operator regions of the *E*. *coli* tryptophan biosynthetic pathway (Yanofsky, C., J. Bacteriol., 158:1018-1024 (1984)) and the left-hand promoter of phage λ (the pLλ promoter, Herskowitz, I. and Hagen, D., Ann. Rev. Genet., 14:399-445 (1980)) may be used as regulatory regions. Meanwhile, vectors applicable to the present disclosure may be constructed by manipulating plasmids commonly used in the art (e.g., pGL3/Luc, pBABE, pSK349, pSC101, ColE1, pBR322, pUC8/9, pHC79, pGEX series, pET series, and pUC19), phages (e.g., λgt•λ4B, A-Charon, λ△z1, and M13), or viruses (e.g., SV40).

The vector may additionally carry a reporter molecule, such as a gene encoding luciferase or β-glucuronidase. The vector may be, but is not limited to, a FLAG vector, a YFP vector, or a GFP vector.

The present disclosure provides an isolated host cell comprising the expression vector.

The host cell of the present disclosure includes not only cells capable of transiently expressing the vector of the present disclosure by transiently transforming or transfecting the vector of the present disclosure, but also stable cell lines capable of stably and continuously expressing the vector of the present disclosure. A stable cell line refers to a cell line in which a specific gene is integrated into the genome and maintained without loss over multiple generations of cell division and proliferation, such that the gene is continuously inherited and expressed.

In addition, any host cell known in the art can be used as a host cell or cell line capable of stably and continuously cloning and expressing the vector of the present disclosure, and examples thereof include *E. coli* strains such as *E. coli* Origami2, *E. coli* JM109, *E. coli* BL21(DE3), *E. coli* RR1, *E. coli* LE392, *E. coli* B, *E. coli* X1776, and *E. coli* W3110; *Bacillus* species such as *Bacillus subtilis* and *Bacillus thuringiensis;* and enterobacterial strains such as *Salmonella typhimurium, Serratia marcescens,* and various *Pseudomonas* species.

Furthermore, when a eukaryotic cell is transformed or transfected with the vector of the present disclosure, host cells such as yeast *(Saccharomyce cerevisiae),* insect cells, and animal cells (e.g., HCT116, HT-29, SW480, HEK293, U2OS, CHO (Chinese hamster ovary), W138, BHK, COS-7, HepG2, 3T3, RIN, and MDCK cell lines) may be used.

In the present disclosure, the terms "transformation" and "transfection" refer to the introduction of a gene into a host cell followed by its expression. The method of transforming or transfecting a vector comprising a target gene into a host cell includes any method of introducing a nucleotide into the cell and may be performed using any appropriate standard technique known in the art.

The method of transporting the vector of the present disclosure into a host cell, when the host cell is a prokaryotic cell, may be carried out by the CaCl₂ method (see., Cohen, S.N. et al., Proc. Natl. Acac. Sci. USA, 9:2110-2114 (1973)), the Hanahan method (see., Cohen, S.N. et al., Proc. Natl. Acac. Sci. USA, 9:2110-2114 (1973); and Hanahan, D., J. Mol. Biol., 166:557-580 (1983)), and the electroporation method (see., Dower, W.J. et al., Nucleic. Acids Res., 16:6127-6145 (1988)), etc. In addition, when the host cell is a eukaryotic cell, the vector may be introduced into the host cell by microinjection (see., Capecchi, M.R., Cell, 22:479(1980)), calcium phosphate precipitation (see., Graham, F.L. et al., Virology, 52:456(1973)), electroporation (see., Neumann, E. et al., EMBO J., 1:841(1982)), liposome-mediated transfection (see., Wong, T.K. et al., Gene, 10:87(1980)), DEAE-dextran treatment (see., Gopal, Mol. Cell Biol., 5:1188-1190(1985)), and gene bombardment (see., Yang et al., Proc. Natl. Acad. Sci., 87:9568-9572(1990)), etc.

The present disclosure provides a method for producing a nucleic acid with enhanced protein expression efficiency.

The method for producing the nucleic acid includes a step of linking a fragment of the 5'UTR of RAC1 to the 5' end of an RNA sequence encoding a target protein and a fragment of the 3'UTR of β-catenin to the 3' end thereof.

In the present disclosure, the "a step of linking" does not simply refer to the physical joining of individual nucleic acid fragments, but also encompasses a process for producing a nucleic acid sequence encoding a target protein, in which the 5'UTR of RAC1 and the 3'UTR of β-catenin, or a fragment thereof, are linked to the 5' and 3' ends, respectively, through transcription from a DNA molecule.

The nucleic acid may be produced through in vitro transcription.

Further, a construct comprising a DNA sequence corresponding to or complementary to the nucleotide sequence of the nucleic acid molecule may be incorporated into a vector for storage or utilization.

For example, in an embodiment, the vector itself may be transformed into a cell. In this case, the nucleic acid construct may be stably or permanently transformed, and the target peptide may be transcribed and translated from the vector using the transcription and translation systems of the transformant, thereby expressing the target protein or peptide.

Meanwhile, in another embodiment, a nucleic acid may be produced through in vitro transcription using the vector as a template, and then transformed into a cell. In this case, the nucleic acid may be transfected temporarily, and the target protein or peptide may be translated using the translation system of the transformant, resulting in expression of the target protein or peptide. In the case of a nucleic acid obtained by in vitro transcription using the above vector as a template, the presence of a poly(A) sequence downstream of the sequence encoding the target protein or peptide allows the nucleic acid to remain highly stable within the cytoplasm even after introduction into a cell, while enables long-term expression of the target protein or peptide.

Therefore, another aspect of the present disclosure is to provide a method for producing a nucleic acid with improved stability, comprising performing in vitro transcription using the vector as a template.

In vitro transcription refers to the process of synthesizing RNA in a test tube using RNA polymerase, its cofactor, substrate NTPs, and template DNA, which is widely known to those skilled in the art. Therefore, those skilled in the art may perform the above process using an appropriate method.

In particular, in the method for producing the nucleic acid of the present disclosure, the nucleic acid construct comprised in the vector comprises a sequence corresponding to or complementary to the poly(A) sequence, such that in vitro transcription as described above is sufficient to produce the nucleic acid, and separate or additional in vitro poly(A) tailing may not be performed after in vitro transcription. In the present disclosure, "in vitro transcription (IVT)" refers to DNA-dependent RNA synthesis outside of a cell or in vitro. The template DNA may be linearized with a suitable restriction enzyme prior to in vitro transcription. Reagents used for RNA in vitro transcription typically include, but are not limited to, a polymerase, such as bacteriophage-encoded RNA polymerases (T7, T3, SP6, or Syn5); ribonucleotide triphosphates (NTPs) for four bases (adenine, cytosine, guanine, and uracil); and optionally, cap analogs; modified nucleotides; and RNase inhibitors.

Specifically, the nucleic acid may be produced by in vitro transcription using the vector according to the present disclosure as a template, and then may be introduced into cells by transfection. In this case, the nucleic acid may be transfected temporarily, and the target protein may be translated and expressed using the translation system of the transformant. In other words, a method for producing a nucleic acid through in vitro transcription using a recombinant vector as a template may also be provided.

According to an embodiment, the nucleic acid construct according to the present disclosure comprises a poly(A) tail downstream of the coding sequence encoding the target protein. As a result, the nucleic acid remains highly stable in the cytoplasm even after introduction into a cell, enabling long-term expression of the target protein. Therefore, there may be provided a method for producing a nucleic acid with enhanced stability by performing in vitro transcription using the vector as a template. In other words, by incorporating a sequence corresponding to or complementary to the poly(A) tail in the gene construct comprised within the above vector, a stable nucleic acid of uniform length may be produced solely through the above-described in vitro transcription, without the need for separate or additional in vitro poly(A) tailing.

The present inventors have confirmed that when a nucleic acid comprising the 5'UTR of RAC1 or a fragment thereof and the 3'UTR of β-catenin or a fragment thereof is synthesized in a cell via a DNA plasmid, or when the nucleic acid synthesized through in vitro transcription is used, the expression efficiency of the target protein is increased, and the expression pattern within the cell can be modulated accordingly.

Since the nucleic acid production method of the present disclosure employs the nucleic acid construct with enhanced expression efficiency described in another aspect of the invention, all overlapping features between the two aspects are equally applicable, and the detailed description thereof is omitted here to avoid unnecessary complexity in the present specification.

When the nucleic acid construct with enhanced protein expression efficiency of the present disclosure, the expression vector comprising the same, the transformant, the host cell, and the composition are used, the expression of the desired protein may be effectively enhanced.

The present disclosure also provides a composition comprising the nucleic acid construct and/or the vector.

The present disclosure also provides a pharmaceutical composition comprising the nucleic acid construct and/or the vector.

Another aspect of the present disclosure is to provide a composition comprising the nucleic acid construct and/or the vector; and a pharmaceutically acceptable carrier.

Another aspect of the present disclosure is to provide a pharmaceutical composition comprising the nucleic acid construct and/or the vector; and a pharmaceutically acceptable carrier.

The pharmaceutical composition of the present disclosure may include a pharmaceutically acceptable carrier and may be formulated, using conventional methods, into oral dosage forms such as powders, granules, tablets, capsules, suspensions, emulsions, syrups, and aerosols, as well as topical preparations, suppositories, and sterile injectable solutions. The most preferred dosage form of the pharmaceutical composition is a sterile injectable solution.

When the pharmaceutical composition of the present disclosure is formulated into an oral solid preparation, it may include tablets, pills, powders, granules, capsules, etc. These solid preparations may include at least one excipient, such as starch, calcium carbonate, sucrose, lactose, gelatin, etc., as well as lubricants such as magnesium stearate and talc, but are not limited thereto.

When the pharmaceutical composition of the present disclosure is formulated as an oral liquid, it includes a suspension, a solution, an emulsion, a syrup, etc., and includes, but is not limited to, a diluent such as water or liquid paraffin, a wetting agent, a sweetener, a fragrance, a preservative, etc.

When the pharmaceutical composition of the present disclosure is formulated for parenteral use, it includes sterile aqueous solutions, non-aqueous solvents, suspensions, emulsions, lyophilized preparations, and suppositories. The non-aqueous solvent and the suspension include, but are not limited to, propylene glycol, polyethylene glycol, vegetable oils such as olive oil, and injectable esters such as ethyl oleate. The suppository bases may include, but are not limited to, witepsol, macrogol, Tween 61, cacao butter, laurin butter, and glycerogelatin.

The pharmaceutical composition may be administered singly or in multiple doses in a pharmaceutically effective amount. The term "pharmaceutically effective amount" as used herein refers to an amount sufficient to prevent or treat a disease at a reasonable benefit/risk ratio applicable to medical immunostimulation, particularly when administered in combination with a therapeutic agent, without compromising the efficacy of the therapeutic agent. The effective dosage level may be determined in consideration of factors such as the severity of the condition, the activity of the drug, the patient's age, weight, health status, and sex, the patient's sensitivity to the drug, the time of administration of the composition of the present disclosure used, the route of administration and excretion rate, the duration of treatment, drugs combined with or concurrently used with the composition of the present disclosure used, and other factors well known in the medical field.

The pharmaceutical composition of the present disclosure may be administered to mammals, such as rats, mice, livestock, and humans, via various routes, including, but not limited to, oral administration, intrathecal, intra-auricular, intraperitoneal, intravenous, intramuscular, subcutaneous, intrauterine, sublingual, or intracerebrovascular injection.

The pharmaceutical composition of the present disclosure may comprise 0.01 to 95% by weight, preferably 1 to 80% by weight, of the nucleic acid construct and/or vector, based on the total weight of the composition.

The nucleic acid construct and/or vector of the present disclosure is formulated to be introduced into mammalian cells and to be expressed therein. These compositions may be particularly useful for the treatment and/or prevention of diseases. Numerous methods may be used to express the nucleic acid construct and/or vector within a host cell, and any suitable method may be used.

For example, the nucleic acid construct and/or vector according to the present disclosure may be inserted into viral vectors such as encephalomyocarditis virus (EMCV), adenovirus, adeno-associated virus, retrovirus, vaccinia, or other poxviruses (e.g., avian pox virus).

According to an exemplary embodiment, the nucleic acid construct described above may be inserted into a suitable vector and then transformed into a nucleic acid molecule in RNA form through in vitro transcription (IVT).

Many vectors available and known in the art may be used for the purposes of the present disclosure. The selection of an appropriate vector will primarily depend on the size of the nucleic acid molecule inserted into the vector and the specific host cell being transformed with the vector.

The pharmaceutical composition further comprises a lipid nanoparticle (LNP), a polymeric microparticle, and an oil-in-water emulsion. For example, polynucleotides, RNA, cRNA, or self-replicating RNA are encapsulated in, bound to, or adsorbed onto LNPs, polymeric microparticles, and oil-in-water emulsions. In one example, the polynucleotide is encapsulated in, bound to, or adsorbed onto LNPs, polymeric microparticles, and oil-in-water emulsions. In another example, RNA is encapsulated in, bound to, or adsorbed onto LNPs, polymeric microparticles, and oil-in-water emulsions.

In one example, the pharmaceutical composition further comprises an LNP. For example, the nucleic acid construct is encapsulated in the LNP. In another example, the RNA is encapsulated in the LNP. In another example, the RNA is adsorbed onto the LNP.

In one example, the LNP comprises a PEG-lipid, a structural lipid, and/or a neutral lipid. For example, the LNP comprises a PEG-lipid, a structural lipid, and/or a neutral lipid. In another example, the LNP comprises a PEG-lipid, a structural lipid, and/or a neutral lipid. In one example, the LNP further comprises a cationic lipid. In another example, the LNP does not comprise a cationic lipid.

In one example, the pharmaceutical composition further comprises polymeric microparticles. For example, the polynucleotide is encapsulated in the polymeric microparticles. In another example, the RNA is encapsulated in the polymeric microparticles. In another example, the RNA is bound to the polymeric microparticles. In another example, the RNA is adsorbed onto the polymeric microparticles.

In one example, the pharmaceutical composition further comprises an oil-in-water emulsion. For example, the polynucleotide is encapsulated in the oil-in-water emulsion. In another example, the RNA is encapsulated in the oil-in-water emulsion. For example, the RNA is bound to the oil-in-water emulsion.

The present disclosure provides a method for preventing or treating a disease or condition in a subject, comprising administering a pharmaceutical composition of the present disclosure to a subject in need of administration.

The present disclosure provides the use of a pharmaceutical composition of the present disclosure in the preparation of a medicament for preventing or treating a disease or condition in a subject in need thereof.

The present disclosure provides a method for inducing an immune response in a subject, comprising administering a pharmaceutical composition of the present disclosure to the subject in need of administration.

The present disclosure also provides the use of the pharmaceutical composition of the present disclosure in the preparation of a medicament for inducing an immune response in a subject in need thereof.

The term "subject" of the present disclosure includes an animal or human whose symptoms can be improved by administration of the pharmaceutical composition of the present disclosure.

The term "administration" in the present disclosure refers to the introduction of a given substance into a human or animal by any suitable method. The pharmaceutical composition of the present disclosure may be administered orally or parenterally via any conventional route, provided that it can reach the target tissue. Furthermore, the pharmaceutical composition according to the present disclosure may be administered by any device capable of transporting the active ingredient to target cells.

The present disclosure also provides an immunogenic composition comprising the nucleic acid construct, vector, and/or pharmaceutical composition for use as a vaccine.

For example, administration of the composition elicits a humoral and/or cell-mediated immune response. In one example, the composition induces a humoral immune response in the subject. For example, the humoral immune response is an antibody-mediated immune response. In another example, the composition induces a cell-mediated immune response. For example, the cell-mediated immune response induces the activation of antigen-specific cytotoxic T cells.

Such immunogenic compositions may further include an adjuvant.

The term "adjuvant" as used herein generally refers to any substance that increases the humoral or cellular immune response to an antigen. The adjuvants are used to achieve two purposes: a purpose of slowing the release of the antigen from the injection site and a purpose of stimulating the immune system.

Immunostimulants include protamine, nucleolin, spermine, spermidine and cationic polysaccharides, stabilized cationic peptides or polypeptides, in particular chitosan, TDM, MDP, muramyl dipeptide, pluronic, alum solution, aluminum hydroxide, ADJUMER (polyphosphazene); aluminum phosphate gel; glucans of algae; algammulin; aluminum hydroxide gel (alum); high protein-absorbing aluminum hydroxide gel; low viscosity aluminum hydroxide gel; AF or SPT (emulsion of squalane (5%), Tween-80 (0.2%), PLURONIC-L121 (1.25%), phosphate-buffered saline, pH 7.4); AVRIDINE (propanediamine); BAY R1005 ((N-(2-deoxy-2-L-leucylamino-b-D-glucopyranosyl)-N-octadecyldodecanoyl-amide hydroacetate); CALCITRIOL (1α,25-dihydroxy-vitamin D3); calcium phosphate gel; calcium phosphate nanoparticles (CAPTM); cholera holotoxin, cholera-toxin-A1-protein-A-D-segment fusion protein, subunit B of cholera toxin; CRL 1005 (block copolymer P1205); cytokine-loaded liposomes; dimethyldioctadecylammonium bromide (DDA); dehydroepiandrosterone (DHEA); dimyristoyl phosphatidylcholine (DMPC); dimyristoyl phosphatidylglycerol (DMPG); DOC/alum complex (deoxycholic acid sodium salt); Freund's complete adjuvant; Freund's incomplete adjuvant; gamma inulin; Gerbu's adjuvant (a mixture of N-acetylglucosaminyl-(p1-4)-N-acetylmuramyl-L-alanyl-D-glutamine (GMDP), dimethyldioctadecylammonium chloride (DDA), and zinc-L-proline complex (ZnPro-8); GM-CSF); N-acetylglucosaminyl-(β1-4)-N-acetylmuramyl-L-alanyl-D-isoglutamine (GMDP); Imiquimod (1-(2-methylpropyl)-1H-imidazo[4,5-c]quinolin-4-amine); ImmTher (N-acetylglucosaminyl-N-acetylmuramyl-L-Ala-D-isoGlu-L-Ala-glycerol dipalmitate); DRV (immunoliposomes prepared from dehydration-rehydration vesicles); interferon-gamma; interleukin-1beta; interleukin-2; interleukin-7; interleukin-12; ISCOMS ("Immunostimulating Complexes"); ISCOPREP 7.0.3.; liposomes; LOXORIBINE (7-allyl-8-oxoguanine (guanine)); LT oral adjuvant (E. *coli* labile enterotoxin-protoxin); microspheres and microparticles of the composition; MF59; (squalene-water emulsion); MONTANIDE ISA 51 (purified incomplete Freund's adjuvant); MONTANIDE ISA 720 (metabolizable oil adjuvant); MPL (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE (Nac-Mur-L-Ala-D-Gln-OCH3); MURAPALMITINE and D-MURAPALMITINE (Nac-Mur-L-Thr-D-isoGln-sn-glyceroldipalmitoyl); neuraminidase-galactose oxidase (NAGO); nanospheres or nanoparticles of the composition; nonionic surfactant vehicle (NISV); PLEURAN (beta-glucan); PLGA, PGA, and PLA (homopolymers and copolymers of lactic acid and glycolic acid; microspheres/nanospheres); PLURONIC L121; polymethyl methacrylate (PMMA); proteinoid microspheres (PODDS); polyethylene carbamate derivatives;Poly-rA: poly-rU (polyadenylic acid-polyuridylic acid complex); polysorbate 80 (Tween 80); protein cochleate (Avanti Polar Lipids, Inc., Alabaster, AL); STIMULON (QS-21); Quil-A (Quil-A saponin); S-28463 (4-amino-otec-dimethyl-2-ethoxymethyl-1H-imidazo[4,5-c]-quinoline-1-ethanol); SAF-1 ("Syntex adjuvant preparation"); Sendai proteoliposomes and Sendai-containing lipid matrices; Span-85 (sorbitan trioleate); Specol (emulsion of Marcol 52, Span 85, and Tween 85); Squalene or Robane (2,6,10,15,19,23-hexamethyltetracosane and 2,6,10,15,19,23-hexamethyl-2,6,10,14,18,22-tetracosahexane); Stearyltyrosine (octadecyltyrosine hydrochloride); Theramid (N-acetylglucosaminyl-N-acetylmuramyl-L-Ala-D-isoGlu-L-Aladipalmitoxypropylamide); Threonyl-MDP (Termurtide or [thr-1]-MDP; N-acetylmuramyl-L-threonyl-D-isoglutamine); Ty particles (Ty-VLPs or virus-like particles); Walter-Reed liposomes (liposomes comprising lipid A adsorbed onto aluminum hydroxide), and related equivalents.

The present disclosure may also be utilized to provide a platform for enhancing translation efficiency, such as in vaccine production and DNA- or RNA-based protein production. For example, the nucleic acid construct according to the present disclosure or a pharmaceutical composition comprising the same may be employed to induce the expression of antigen proteins, therapeutic proteins, or diagnostic marker proteins.

### [Advantageous Effects]

The 5'UTR sequence according to the present disclosure and the nucleic acid sequence comprising the same, may be employed to effectively enhance the expression of a target protein. This can be accomplished not only through transformation and transfection using DNA plasmids, but also with nucleic acids synthesized through in vitro transcription. The 5'UTR sequence may be utilized in mRNA vaccines and may be applied to the production of therapeutic proteins for specific diseases by incorporating the sequence into various genes to synthesize DNA and RNA constructs.

### [Description of Drawings]

FIG. 1 shows the translation efficiency analysis of DNA and IVT-RNA produced to comprise the 5'UTRs of various genes, through a luciferase reporter assay:
FIG. 1A shows the sequence length (expressed in nt) of the 5'UTR regions of various genes;
FIG. 1B shows RNA produced and purified, comprising the 5'UTR regions of various genes;
FIG. 1C shows the translation efficiency confirmed after transfection of HEK293 cells with DNA comprising the 5'UTR regions of various genes; and
FIG. 1D shows the translation efficiency confirmed after transfection of HEK293 cells with IVT-RNA comprising the 5'UTR regions of various genes.
FIG. 2 shows the translation efficiency confirmed after transfection of SW480 cells with IVT-RNA comprising the 5'UTR regions of various genes.
FIG. 3 shows the translation efficiency analysis of DNA and IVT-RNA produced by comprising the full-length sequence and fragments of the RAC1 5'UTR region, through a luciferase reporter assay:
FIG. 3A shows fragmentation based on a stable stem-loop structure discovered through RNA structural analysis from the RAC1 5'UTR region;
FIG. 3B shows RNA produced and purified, comprising the full-length sequence (FL) and fragments (SL1, SL2, SL3, and combinations thereof) of the RAC1 5'UTR region;
FIG. 3C shows the translation efficiency analysis after transfection of HEK293 cells with DNA comprising the full-length sequence (FL) and fragments (SL1, SL2, SL3, and combinations thereof) of the RAC1 5'UTR region; and
FIG. 3D shows the translation efficiency analysis after transfection of HEK293 cells with IVT-RNA comprising the full-length sequence (FL) and fragments (SL1, SL2, SL3, and combinations thereof) of the RAC1 5'UTR region.
FIG. 4 shows the results of the translation efficiency analysis of constructs for the RAC1 5'UTR and various 3'UTR combinations using a DNA luciferase reporter.
FIG. 5 shows the results of the translation efficiency analysis of constructs for the RAC1 5'UTR and various 3'UTR combinations using the IVT-RNA luciferase reporter.
FIG. 6 shows the comparative results of the translation efficiency analysis of constructs for various 5'UTR and 3'UTR fragments using the IVT-RNA luciferase reporter:
FIG. 6A shows RNA loading results of IVT-RNAs produced with various combinations of 5'UTR and 3'UTR fragments; and
FIG. 6B shows comparison of luciferase expression levels when IVT-RNAs produced with various combinations of 5'UTR and 3'UTR fragments were introduced into HEK293T and HeLa cells.
FIG. 7 shows the results of the translation efficiency of constructs comprising the fragment of RAC1 5'UTR (SL3) and the fragment of CTNNB1 3'UTR using a DNA luciferase reporter.
FIG. 8 shows the results of the translation efficiency of constructs comprising the fragment of RAC1 5'UTR (SL3) and the fragment of CTNNB1 3'UTR using an IVT-RNA luciferase reporter.
FIG. 9A shows a process of predicting a G-quadruplex structure in the RAC1 5'UTR; and FIG. 9B shows a process of substituting guanine (G) with adenine (A) in the G-rich sequence of the Gq2 region, which showed a high G-score.
FIG. 10 shows the results of the translation efficiency analysis of constructs comprising a RAC1 5'UTR mutant sequence using a DNA luciferase reporter:
FIG. 10A shows the structures of mutants (GA1, GA2, GA3) in which the GGG motif in the G-quadruplex sequence of the RAC1 5'UTR is sequentially substituted with adenine (A); and
FIG. 10B shows the comparative results of the luciferase expression levels in HEK293 cells when each mutant was expressed in DNA form, and
FIG. 11 shows the results of the translation efficiency analysis of constructs comprising the RAC1 5'UTR mutant sequence and the CTNNB1 3'UTR combinations using a DNA luciferase reporter.

### [Best Mode]

Hereinafter, the preferred Examples are presented to help understanding of the present disclosure. These following exemplary embodiments are only provided to more easily understand the present disclosure, and the content of the present disclosure is not limited by these Examples.

### Experimental Example 1. Production of Luciferase Reporter

Luciferase reporters were produced to analyze the translation efficiency of various genes employing the 5'UTR.

Specifically, Renilla luciferase clones comprising the 5'UTR sequences listed in Table 1 below were provided by Dr. Stephen Floor (Parnassus campus of the University of California, San Francisco (UCSF)) (Calviello et al Nucleic Acids Res. 2021 May 21;49(9) :5336-5350. doi: 10.1093/nar/gkab287), and a luciferase reporter comprising the human β-globin (HBB) 5'UTR sequence was provided by Dr. Seong-Ryong Kim (Sogang University, Korea). Further, the 5'UTR (Moderna) sequence of the COVID-19 virus was obtained by gene synthesis, and the PDCD4 isol/iso2-derived 5'UTR sequence was obtained as cDNA by RT-PCR from SW480 cells, thereby producing a luciferase reporter. Meanwhile, the 5'UTR sequence derived from the pUC vector was extracted from an *E. coli* vector and obtained by gene synthesis, serving as a control.

**[Table 1]**

| **Gene** | **Sequences** |
|---|---|
| CCNE1_ENS T00000262 | GAGGGGCTGGGAGCCGCGGCGGGGCGGTGCGAGGGCGGGCCGGGGCCGGT TCCGCGCGCAGGGATTTTAAATGTCCCGCTCTGAGCCGGGCGCAGGAGCA |
| 643 | GCCGGCGCGGCCGCCAGCGCGGTGTAGGGGGCAGGCGCGGATCCCGCCAC CGCCGCGCGCTCGGCCCGCCGACTCCCGGCGCCGCCGCCGCCACTGCCGT CGCCGCCGCCGCCTGCCGGGACTGGAGCGCGCCGTCCGCCGCGGACAAGA CCCTGGCCTCAGGCCGGAGCAGCCCCATC (SEQ ID NO: 1) |
| ODC1_ENST 000002341 11 | GACGTCGGCCCGCCGGCGCCCCACCAGCTCCGCGCGGGCCCGGGTTGGCC ACCGCCGGGCCCCCGCCCCTCCCCCGGCGGTGTCCCGGCCGGAACCGATC GTGGCTGGTTTGAGCTGGTGCGTCTCCATGGCGACCCGCCGGTGCTATAA GTAGGGAGCGGCGTGCCGTGGGGCTTTGTCAGTCCCTCCTGTAGCCGCCG CCGCCGCCGCCCGCCGCCCCTCTGCCAGCAGCTCCGGCGCCACCTCGGGC CGGCGTCTCCGGCGGGCGGGAGCCAGGCGCTGACGGGCGCGGCGGGGGCG GCCGAGCGCTCCTGCGGCTGCGACTCAGGCTCCGGCGTCTGCGCTTCCCC ATGGGGCTGGCCTGCGGCGCCTGGGCGCTCTGAGATTGTCACTGCTGTTC CAAGGGCACACGCAGAGGGATTTGGAATTCCTGGAGAGTTGCCTTTGTGA GAAGCTGGAAATATTTCTTTCAATTCCATCTCTTAGTTTTCCATAGGAAC ATCAAGAAATC (SEQ ID NO: 2) |
| RAC1_ENST 000003561 42 | AGTTTTCCTCAGCTTTGGGTGGTGGCCGCTGCCGGGCATCGGCTTCCAGT CCGCGGAGGGCGAGGCGGCGTGGACAGCGGCCCCGGCACCCAGCGCCCCG CCGCCCGCAAGCCGCGCGCCCGTCCGCCGCGCCCCGAGCCCGCCGCTTCC TATCTCAGCGCCCTGCCGCCGCCGCCGCGGCCCAGCGAGCGGCCCTG (SEQ ID NO: 3) |
| HBB | ACATTTGCTTCTGACACAACTGTGTTCACTAGCAACCTCAAACAGAC (SEQ ID NO: 4) |
| COVID rna) (Moderna) | GAATAAACTAGTATCTCTGGTCCCCACAGACTCAGAGAGAACCCGCCACC (SEQ ID NO: 5) |
| PDCD4 iso1 | AGAGACTCTCCGAGGCGGCGGCAGAGACAGAAGAGCGGGGTCGGGGCCGG CTGACCAGGAACCTGGGCGAGCAGCGGCGGGGGCCCGAGGGATTCTGAAG GAAGATTTCCATTAGGTAATTTGTTTAATCAGTGCAAGCGAAATTAAGGG AAA (SEQ ID NO: 6) |
| PDCD4 iso2 | GCCTCGGCCAATAAGCGCCGCCCTCTCGCCCCCGTGTTACTGGGTAGAAG AAAACAAAAACAAACAGAGCGAGAAGGGCCAGAGACTCTCCGAGGCGGCG GCAGAGACAGAAGAGCGGGGTCGGGGCCGGCTGACCAGGAACCTGGGCGA GCAGCGGCGGGGGCCCGAGGGATTCTGAAGGAAGATTTCCATTAGGTAAT TTGTTTAATCAGTGCAAGCGAAATTAAGGGAAA (SEQ ID NO: 7) |
| pUC 5'UTR (Standard *E. coli* vector) | TAGATCCTTTTAAATTAAAAATGAAGTTTTAAATCAATCTAAAGTATATA (SEQ ID NO: 8) |

To synthesize in vitro transcribed RNA (IVT RNA), the above clone, comprising a T7 promoter from pUC57 and 100 adenosine residues downstream of a stop codon to mimic polyadenylation, was cleaved with HIndIII and SalI to remove Renilla luciferase, followed by insertion of firefly luciferase for cloning.

Specifically, the 5'UTR sequence was amplified by polymerase chain reaction (PCR) using the primers listed in Table 2 below, followed by digestion with HindIII and NcoI restriction enzymes. The digested PCR product was cloned into the upstream region of the firefly luciferase gene in the pUC57-T7-Fluc-poly(A)x100 vector and the pGL3/Luc vector, respectively.

**[Table 2]**

| **Gene** | **Forward (5'→3')** | **UTR Reverse (5'→3')** |
|---|---|---|
| CCNE1 | GGGAAGCTTGAGGGCTGGGAGCC GCGGC (SEQ ID NO: 9) | CCCCCATGGATGGGCTGCTCCG GCCTG (SEQ ID NO: 10) |
| ODC1 | GGGAAGCTTGACGTCGGCCCGCCG GCGCC (SEQ ID NO: 11) | CCCAAGCTTGATTTCTTGATGTTC CTATG (SEQ ID NO: 12) |
| RAC1 | GGGAAGCTTAGTTTTCCTCAGCTT TGGGT (SEQ ID NO: 13) | CCCCCATGGCAGGGCCGCTCGCTG GGC (SEQ ID NO: 14) |
| HBB | GGGAAGCTTACATTTGCTTCTGAC ACAAC (SEQ ID NO: 15) | CCCCCATGGGTCTGTTTGAGGTTG CTAGT (SEQ ID NO: 16) |
| COVID (Moderna) | GGGAAGCTTGAATAAACTAGTATC TC (SEQ ID NO: 17) | CCCCCATGGGGTGGCGGTTCTCT C (SEQ ID NO: 18) |
| PDCD4 | GGGAAGCTTAGAGACTCTCCGAGG CGGCG (SEQ ID NO: 19) | CCCCCATGGTTTCCCTTAATTTCG CTTGC (SEQ ID NO: 20) |
| pUC 5'UTR (Standard *E. coli* vector) | GGGAAGCTTTAGATCCTTTTAAAT TAAAAATGAAGTTTTAAATCAATC TAAAGTATATA (SEQ ID NO: 21) | CCCCCATGGTATATACTTTAGATT GATTTAAAACTTCATTTTTAATTT AAAAGGATCTA (SEQ ID NO: 22) |

### Experimental Example 2. Synthesis of In vitro transcribed RNA (IVT RNA)

For in vitro transcription, each clone comprising the T7 promoter and poly(A) tail was digested with XhoI to obtain purified DNA fragments. RNA was then transcribed using a Capped RNA synthesis kit (NEB, E2050) in the presence of Cap analog (ARCA, NEB #S1411).

To the transcribed RNA, 10X T7 polymerase buffer, 2 mM NTP, 8 mM cap analog, 1 µg DNA template, 5 mM DTT, and 2 µl of T7 RNA polymerase mix were added, and incubated at 37°C for 2 hours. To remove the DNA template, 2 µl of DNase I was added, and the reaction was incubated at 37°C for an additional 15 minutes. The RNA synthesized through the above process was purified using the NucleoSpin RNA Clean-up kit (Macherey-Nagel GmbH & Co.).

### Experimental Example 3. Cell Culture and Transfection

HEK293 cells (human embryonic kidney-derived cells) were cultured in Minimum Essential Medium (Gibco), and SW480 cells (colon and rectal adenocarcinoma cells) were cultured in Roswell Park Memorial Institute (RPMI) 1640 (Welgene). Each medium contained 10% fetal bovine serum (FBS) and 1% antibiotics (Hyclone), and the cells were cultured at 5% CO₂ and 37°C.

Synthesized RNA was transfected using Lipofectamine^{™} MessengerMAX^{™} (Thermofisher, LMRNA008), and DNA was transfected using Lipofectamine^{™} 3000.

### Example 1. Confirmation of enhanced translation efficiency by RAC1 5'UTR

To perform a luciferase reporter assay to measure the translation efficiency of 5'UTR mRNA, HEK293 cells were cultured in 12-well plates and transfected with the synthesized T7-5'UTR-FLuc-poly(A)x100 RNA or 5'UTR DNA for 14 to 24 hours. The transfected cells were harvested by washing with 1× PBS, followed by centrifugation to precipitate the cells (cell down), and subsequently lysed by adding 1× Passive lysis buffer (Promega). The lysed cells were centrifuged at 13,000 rpm for 15 minutes to precipitate (cell down), and only the lysate in the supernatant was transferred to a new tube. Each sample was analyzed using a GLOMAX Navigator luciferase assay kit (Promega). The same experiment was also performed on SW480 cells.

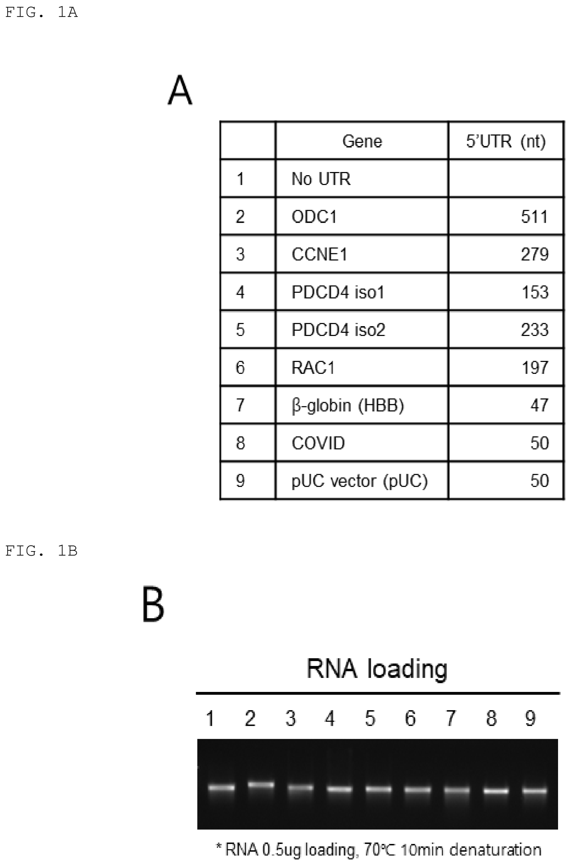

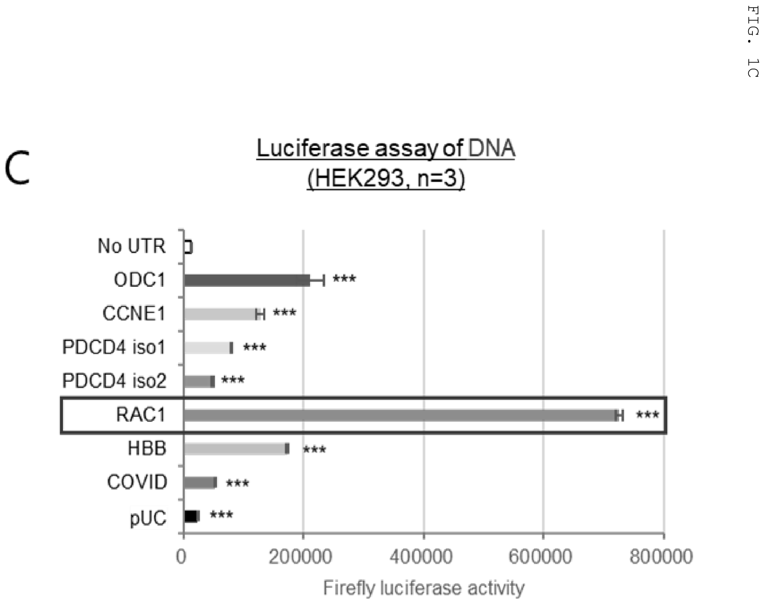

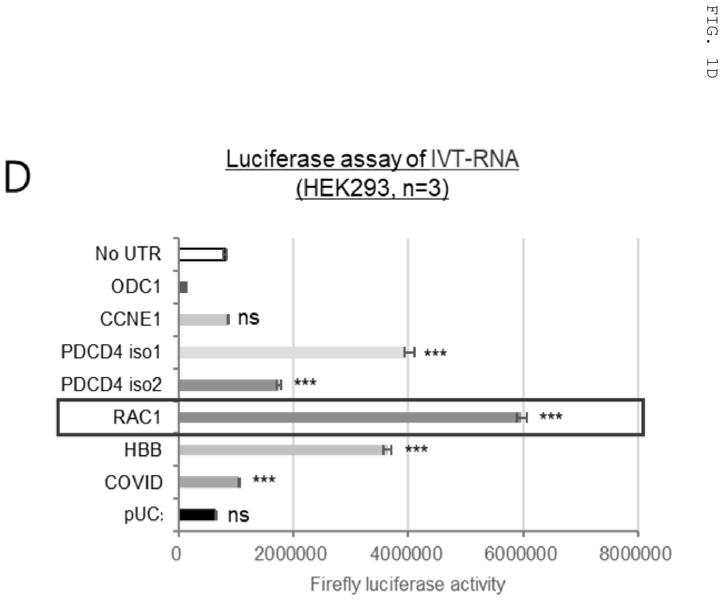

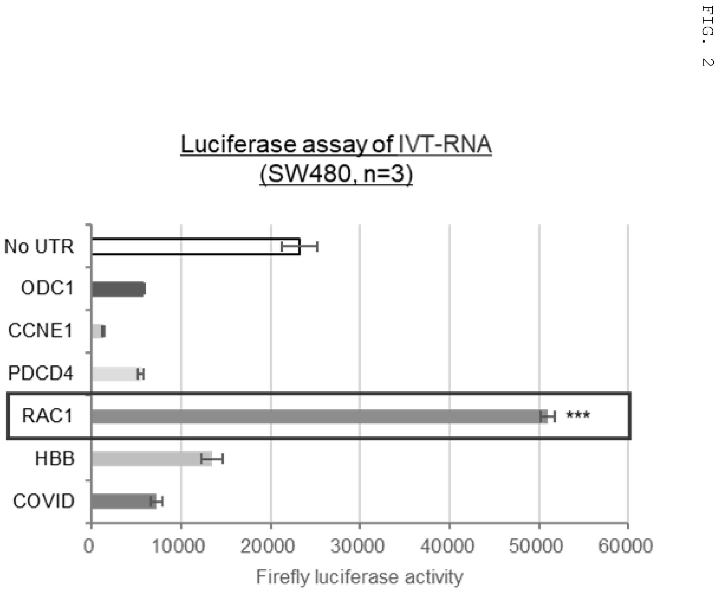

As a result, the inclusion of the RAC1 5'UTR sequence in HEK293 cells transfected with DNA or RNA resulted in a significantly high in translation efficiency (FIGS. 1(C) and 1(D)). Further, the same pattern was observed even when SW480 cells were transfected with RNA (FIG. 2).

Therefore, it was confirmed from the above experiments that the 5'UTR of RAC1 comprises a sequence that effectively increases translation efficiency.

### Example 2. Confirmation of Nucleic Acid Sequences Derived from the RAC1 5'UTR That Enhance Translation Efficiency

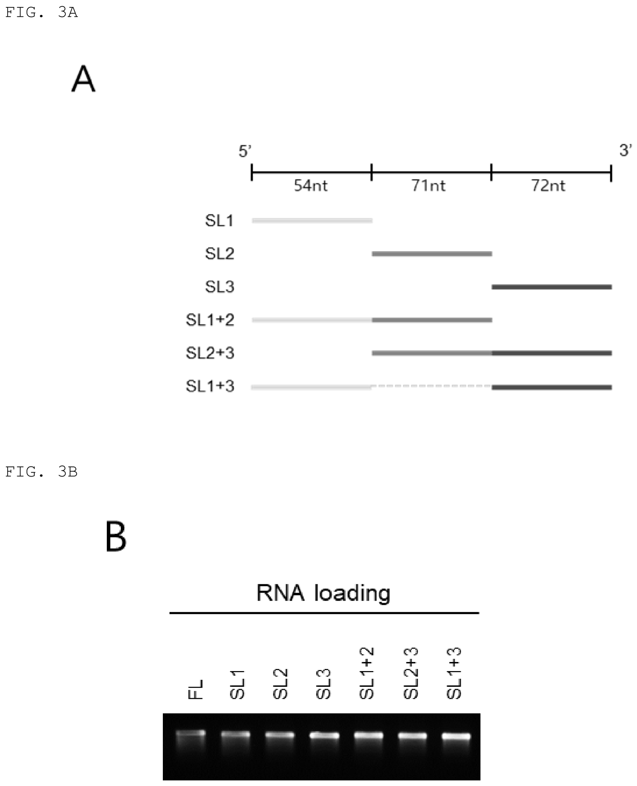

To confirm the exact site of regulatory elements within the RAC1 5'UTR that enhance translation efficiency, RNA structural analysis was performed to identify sequences with the most stable stem-loop structures (FIG. 3A).

These sequences were designated SL1 (stem-loop 1), SL2 (stem-loop 2), and SL3 (stem-loop 3) and are listed in Table 3. The primer sequences for SL1, SL2, and SL3 are listed in Table 4.

**[Table 3]**

| **Name** | **Sequences (5'→3')**) |
|---|---|
| RAC1 5'UTR full-length (FL) | CTGTTTCTCTGCAGTTTTCCTCAGCTTTGGGTGGTGGCCGCTGCCGGGC ATCGGCTTCCAGTCCGCGGAGGGCGAGGCGGCGTGGACAGCGGCCCCGG CACCCAGCGCCCCGCCGCCCGCAAGCCGCGCGCCCGTCCGCCGCGCCCC GAGCCCGCCGCTTCCTATCTCAGCGCCCTGCCGCCGCCGCGGCCCA GCGAGCGGCCCTG (SEQ ID NO: 30) |
| SL1 (54nt) | AGTTTTCCTCAGCTTTGGGTGGTGGCCGCTGCCGGGCATCGGCTTCCAG TCCGC (SEQ ID NO: 31) |
| SL2 (71nt) | GGAGGGCGAGGCGGCGTGGACAGCGGCCCCGGCACCCAGCGCCCCGCCG CCCGCAAGCCGCGCGCCCGTCC (SEQ ID NO: 32) |
| SL3 (72nt) | GCCGCGCCCCGAGCCCGCCGCTTCCTATCTCAGCGCCCTGCCGCCGCCG CCGCGGCCCAGCGAGCGGCCCTG (SEQ ID NO: 33) |
| SL1+2 (125nt) | AGTTTTCCTCAGCTTTGGGTGGTGGCCGCTGCCGGGCATCGGCTTCCAG TCCGCGGAGGGCGAGGCGGCGTGGACAGCGGCCCCGGCACCCAGCGCCC CGCCGCCCGCAAGCCGCGCGCCCGTCC (SEQ ID NO: 34) |
| SL2+3 (143nt) | GGAGGGCGAGGCGGCGTGGACAGCGGCCCCGGCACCCAGCGCCCCGCCG CCCGCAAGCCGCGCGCCCGTCCGCCGCGCCCCGAGCCCGCCGCTTCCTA TCTCAGCGCCCTGCCGCCGCCGCCGCGGCCCAGCGAGCGGCCCTG (SEQ ID NO: 35) |
| SL1+3 (126nt) | AGTTTTCCTCAGCTTTGGGTGGTGGCCGCTGCCGGGCATCGGCTTCCAG TCCGCGCCGCGCCCCGAGCCCGCCGCTTCCTATCTCAGCGCCCTGCCGC CGCCGCCGCGGCCCAGCGAGCGGCCCTG (SEQ ID NO: 36) |

**[Table 4]**

| **Name** | **Sequences (5'→3')** |
|---|---|
| HindIII-RAC1 SL1 Forward | GGGAAGCTTAGTTTTCCTCAGCTTTGGGT (SEQ ID NO: 37) |
| pGL3-RAC1 SL1 Reverse | TGTTTTTGGCGTCTTCCATGGGCGGACTGGAAGCCGATGC (SEQ ID NO: 38) |
| HindIII-RAC1 SL2 Forward | GGGAAGCTTGGAGGGCGAGGCGG (SEQ ID NO: 39) |
| pGL3-RAC1 SL2 Reverse | TGTTTTTGGCGTCTTCCATGGGGACGGGCGCGCGG (SEQ ID NO: 40) |
| HindIII-RAC1 SL3 Forward | GGGAAGCTTGCCGCGCCCCGAGCC (SEQ ID NO: 41) |
| pGL3-RAC1 SL3 Reverse | TGTTTTTGGCGTCTTCCATGGCAGGGCCGCTCGCTGGG (SEQ ID NO: 42) |

RNAs comprising the SL1, SL2, SL3, and their combinations were produced (FIG. 3B), and subjected to a luciferase reporter assay.

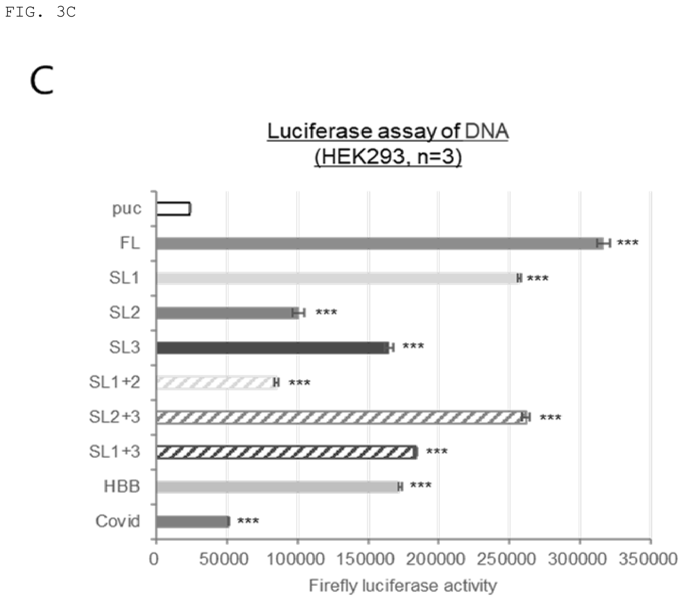

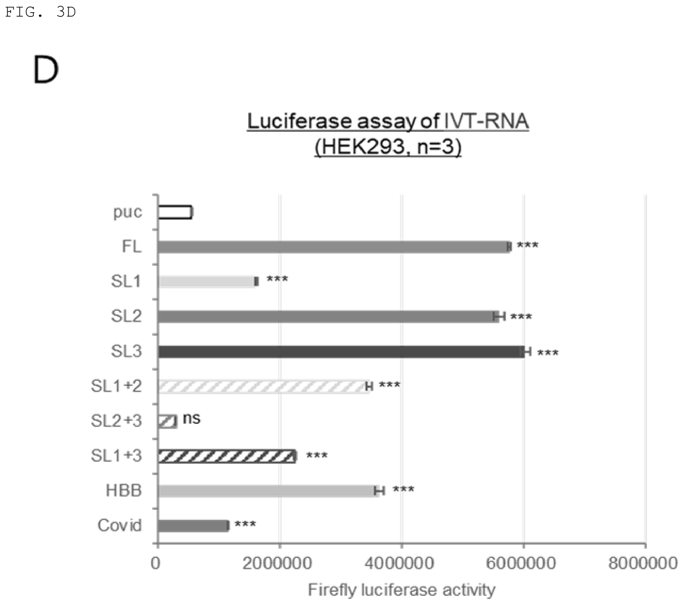

As a result, transfection of DNA with the SL1 and SL2+3 fragments resulted in very high translation efficiencies, comparable to those achieved with the full-length (FL) (FIG. 3C). Meanwhile, for IVT-RNA, the group transfected with the SL2 and SL3 fragments showed higher translation efficiencies than the group transfected with the full-length (FL) (FIG. 3D).

### Example 3. Translation Efficiency Analysis of Combinations of the RAC1 5'UTR and Various Gene-Derived 3'UTRs

To determine whether the RAC1 5'UTR retains its translation-enhancing function when combined with 3'UTRs from various genes, experiments were conducted using 3'UTR fragments from various origins.

Furthermore, to verify 3'UTR fragments that enhance translation efficiency, 3'UTR fragments (F1, F3, and F1+3) derived from CTNNB1 were identified and combined with 5'UTR to construct a luciferase reporter, followed by confirmation of translation efficiency. This was performed by applying the methods described in the Experimental Examples above, and the sequences of the 3'UTR fragment and the primers therefor are shown in Tables 5 and 6, respectively.

**[Table 5]**

| **Name** | **Sequences (5'→3')** |
|---|---|
| F1 | CTGTATTGTCTGAACTTGCATTGTGATTGGCCTGTAGAGTTGCTGAGAGG GCTCGAGGGGTGGGCTGGTATCTCAGAAAGTGCCTGACACACTAACCAAG CTGAGTTTCCTATGGGAACAATTGAAGTAAACTTTTTGTTCTGGTCCTTT TTGGTCGAGGAGTAACAATACAAATGGATTTTGGGAGTGACTCAAGAAGT GAAGAATGCACAAGAATGGATCACAAGATGGAATTTATCAAACCCTAGCC TTGCTTGTTAAATTTTTTTTTTTTTTTTTTTAAGAATATCTGTAATGGTA CTGACTTTGCTTGCTTTGAAGTAGCTCTTTTTTTTTTTTTTTTTTTTTTT TTGCAGTAACTGTTTTTTAAGTCTCTC (SEQ ID NO: 23) |
| F3 | GTAACTGTTTTTTAAGTCTCTCGTAGTGTTAAGTTATAGTGAATACTGCT ACAGCAATTTCTAATTTTTAAGAATTGAGTAATGGTGTAGAACA/CTAAT TCATAATCACTCTAATTAATTGTAATCTGAATAAAGTGTAACAATTGTGT AGCCTTTTTGTATAAAATAGACAAATAGAAAATGGTCCAATTAGTTTCCT TTTTAATATGCTTAAAATAAGCAGGTGGATCTATTTCATGTTTTTGATCA AAAACTATTTGGGATATGTATGGGTAGGGTAAATCAGTAAGAGGTGT (SEQ ID NO: 24) |
| F1+3 | CTGTATTGTCTGAACTTGCATTGTGATTGGCCTGTAGAGTTGCTGAGAGG GCTCGAGGGGTGGGCTGGTATCTCAGAAAGTGCCTGACACACTAACCAAG CTGAGTTTCCTATGGGAACAATTGAAGTAAACTTTTTGTTCTGGTCCTTT TTGGTCGAGGAGTAACAATACAAATGGATTTTGGGAGTGACTCAAGAAGT GAAGAATGCACAAGAATGGATCACAAGATGGAATTTATCAAACCCTAGCC TTGCTTGTTAAATTTTTTTTTTTTTTTTTTTAAGAATATCTGTAATGGTA CTGACTTTGCTTGCTTTGAAGTAGCTCTTTTTTTTTTTTTTTTTTTTTTT TTGCAGTAACTGTTTTTTAAGTCTCTCGTAACTGTTTTTTAAGTCTCTCG TAGTGTTAAGTTATAGTGAATACTGCTACAGCAATTTCTAATTTTTAAGA ATTGAGTAATGGTGTAGAACA/CTAATTCATAATCACTCTAATTAATTGT AATCTGAATAAAGTGTAACAATTGTGTAGCCTTTTTGTATAAAATAGACA AATAGAAAATGGTCCAATTAGTTTCCTTTTTAATATGCTTAAAATAAGCA GGTGGATCTATTTCATGTTTTTGATCAAAAACTATTTGGGATATGTATGG GTAGGGTAAATCAGTAAGAGGTGT (SEQ ID NO: 25) |

**[Table 6]**

| **Name** | **Sequences (5'→3')** |
|---|---|
| CTNNB1 F1 Xbal Forward | CGTGTAATTCTAGACTGTATTGTCTGAACTTGCATT (SEQ ID NO: 26) |
| CTNNB1 F1-A60 Sall Reverse | CATCGGTCGACGGATCCTTTTTTTTTTTTTTTTTTTTTTTTTTTTTTTT TTTTTTTTTTTTTTTTTTTTTTTTTTTTGAGAGACTTAAAA   (SEQ   ID NO: 27) |
| CTNNB1 F3 Xbal Forward | CGTGTAATTCTAGAGTAACTGTTTTTTAAGTCTCTCGT (SEQ ID NO: 28) |
| CTNNB1 F3-A60 Sall Reverse | CATCGGTCGACGGATCCTTTTTTTTTTTTTTTTTTTTTTTTTTTTTTTTTT TTTTTTTTTTTTTTTTTTTTTACACCTCTTACTG   (SEQ  ID NO: 29) |

Further, combination experiments were conducted using 3'UTRs (pUC, β-actin, HBB), which are commonly used as control. In other words, analyses were conducted simultaneously with the pUC vector-derived 3'UTR, the β-actin 3'UTR, and the human β-globin 3'UTR that are known in the art.

Specifically, the full-length RAC1 5'UTR (FL) and the pUC vector-derived 3'UTR (FL+pUC vector), the full-length RAC1 5'UTR and the β-actin 3'UTR (FL+β-actin), and the full-length RAC1 5'UTR and the human β-globin 3'UTR (FL+HBB) combinations were analyzed together.

To analyze the translation efficiency of the RAC1 5'UTR and various 3'UTR combinations, a 3'UTR reporter vector was constructed based on the pGL3 control vector. All 3'UTR sequences were amplified by PCR using the pUC 3'UTR or HBB 3'UTR sequence as a template, with primers designed to include BamHI and SalI restriction enzyme recognition sequences. The β-actin 3'UTR was amplified using cDNA from HCT116 cells as a template, and the resulting PCR amplification product was ligated into the pGL3 vector digested with the same restriction enzyme for subsequent cloning.

The 3'UTR sequences and their corresponding primer sequences are shown in Tables 7 and 8, respectively.

**[Table 7]**

| **Name** | **Sequence (5'→3')** |
|---|---|
| β-actin_3'UTR | GCGGACTATGACTTAGTTGCGTTACACCCTTTCTTGACAAAAC CTAACTTGCGCAGAAAACAAGATGAGATTGGCATGGCTTTATT TGTTTTTTTTGTTTTGTTTTGGTTTTTTTTTTTTTTTTGGCTT GACTCAGGATTTAAAAACTGGAACGGTGAAGGTGACAGCAGTC GGTTGGAGCGAGCATCCCCCAAAGTTCACAATGTGGCCGAGGA CTTTGATTGCACATTGTTGTTTTTTTAATAGTCATTCCAAATA TGAGATGCGTTGTTACAGGAAGTCCCTTGCCATCCTAAAAGCC ACCCCACTTCTCTCTAAGGAGAATGGCCCAGTCCTCTCCCAAG TCCACACAGGGGAGGTGATAGCATTGCTTTCGTGTAAATTATG TAATGCAAAATTTTTTTAATCTTCGCCTTAATACTTTTTTATT TTGTTTTATTTTGAATGATGAGCCTTCGTGCCCCCCCTTCCCC CTTTTTTGTCCCCCAACTTGAGATGTATGAAGGCTTTTGGTCT CCCTGGGAGTGGGTGGAGGCAGCCAGGGCTTACCTGTACACTG ACTTGAGACCAGTTGAATAAAAGTGCACACCTTAAAAATGA (SEQ ID NO: 43) |
| pUC_3'UTR | AGGATACAGCGGCTTCTGCGCGACTTATAAGAGCTCCTTGTGC GGCGCCATTTTAAGCCTCTCGGTCTGTGGCAGCAGCGTTGGCC CGGCCCCGGGAGCGGAGAGCGAGGGGAGGCGGAGACGGAGGAA GGTCTGAGGAGCAGCTTCAGTCCCCGCCGAGCCGCCACCGCAG GTCGAGGACGGTCGGACTCCCGCGGCGGGAGGAGCCTGTTCCC CTGAGGGTATTTGAAGTATACCATACAACTGTTTTGAAAATCC AGCGTGGACA (SEQ ID NO: 44) |
| HBB_3'UTR | GCTCGCTTTCTTGCTGTCCAATTTCTATTAAAGGTTCCTTTGT TCCCTAAGTCCAACTACTAAACTGGGGGATATTATGAAGGGCC TTGAGCATCTGGATTCTGCCTAATAAAAAACATTTATTTTCAT TGC (SEQ ID NO: 45) |

**[Table 8]**

| **Name** | **Sequence (5**'**→3'**) |
|---|---|
| β-actin_3'UTR_ BamHI Forward | CCCGGATCCGCGGACTATGACTTAGTTGCG (SEQ ID NO: 46) |
| β-actin_3'UTR_ SalI_Reverse | GGGGTCGACTCATTTTTAAGGTGTGCACTTTTATTCAACT (SEQ ID NO: 47) |
| pUC_3'UTR_ BamHI Forward | CCCGGATCCAGGATACAGCGGCTTCTGC (SEQ ID NO: 48) |
| pUC_3'UTR_ SalI_Reverse | GGGGTCGACTGTCCACGCTGGATTTTCAAAACA (SEQ ID NO: 49) |
| HBB_3'UTR_ BamHI Forward | CCCGGATCCGCTCGCTTTCTTGCTGTC (SEQ ID NO: 50) |
| HBB_3'UTR_ SalI_Reverse | CCCGCTGACGCAATGAAAATAAATGTTTT (SEQ ID NO: 51) |

Subsequently, the respective RAC1 5'UTR sequences obtained as described above were combined with the 3'UTR sequences of various genes and inserted into the pGL3 reporter vector to construct various 5'UTR and 3'UTR combination reporters.

Meanwhile, the pUC57 vector was used for in vitro transcription (IVT) experiments, and the cloning procedure was performed in the same manner as in the construction of the pGL3-based reporter. The pUC57-based IVT reporter contained the Firefly luciferase coding sequence, flanked upstream by the T7 promoter and downstream by a 100-nt poly(A) sequence. The template DNA for in vitro transcription was linearized by cleavage of the XhoI recognition site located downstream of the 100-nt poly(A) sequence, which was then used for the IVT reaction.

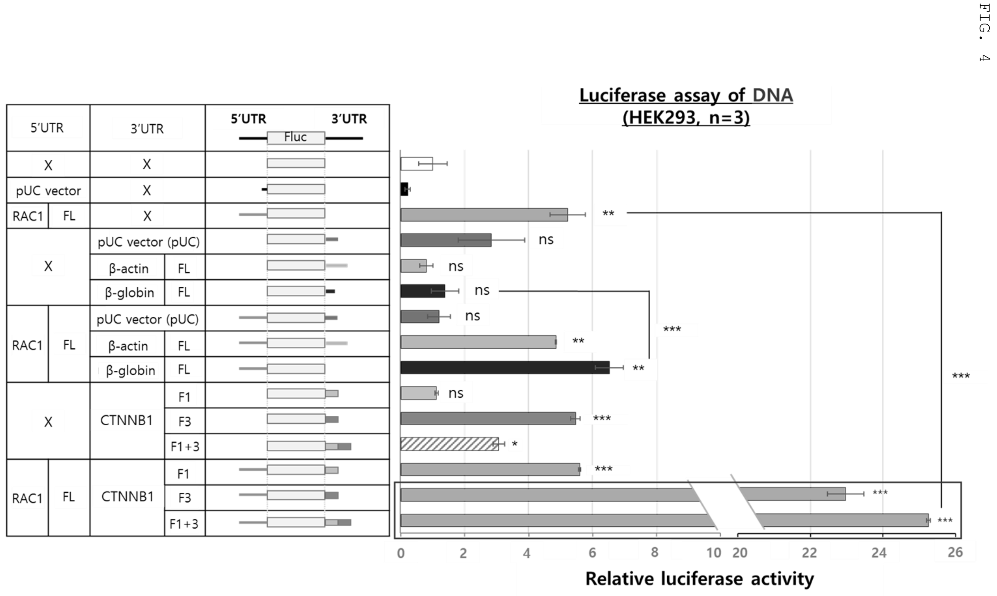

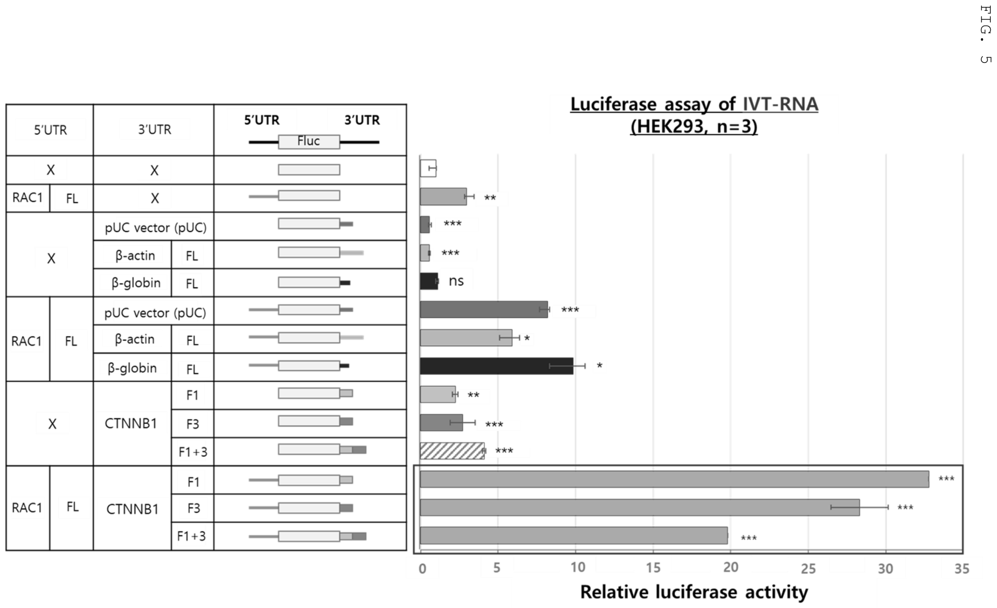

The results are shown in FIGS. 4 and 5.

As shown in FIGS. 4 and 5, when HEK293 cells were transfected with DNA and RNA, respectively, the inclusion of the RAC1 5'UTR sequence resulted in increased translation efficiency in all experimental groups. Notably, the above combination also demonstrated an increase in translation efficiency, and the use of the CTNNB1-derived 3'UTR fragment as the 3'UTR resulted in a particularly significant increase in translation efficiency.

Specifically, for DNA, the highest translation efficiency was achieved when using combinations of FL and F3, and FL and F1+3. Conversely, for RNA, the highest translation efficiency was achieved when using combinations of FL and F1, FL and F3, and FL and F1+3.

Thus, the above experiments confirmed that the RAC1 5'UTR fragment could significantly enhance translation efficiency even when combined with various 3'UTRs.

In particular, the combination of the RAC1 5'UTR with the three CNTNNB1 3'UTR fragments resulted in the most effective enhancement of translation efficiency compared to three types of CNTNNB1 3'UTR fragments individually.

Therefore, it was confirmed that the RAC1 5'UTR is a functional element that provides a stable and consistent translation-enhancing effect even when combined with the 3'UTRs of various genes, not limited to a specific 3'UTR, and is a potent translation-enhancing element that exhibits a synergistic level of increased translation effect with a specific 3'UTR (e.g., the 3'UTR of β-catenin or a fragment thereof).

### Example 4. Confirmation of Translation Efficiency by Combination of 5'UTRs of Various Genes and CTNNB1 3'UTR Fragments

To verify the translation efficiency enhancement effect of the combination of 5'UTRs of various genes and CTNNB1 3'UTR fragments, experiments were conducted using the CTNNB1-derived 3'UTR fragment, the 3'UTR presented in Example 3, along with other 5'UTRs.

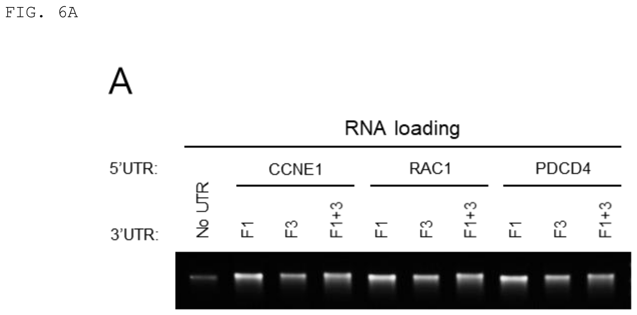

Similar to the previous experimental method, 3'UTR fragments (F1, F3, and F1+3) derived from CTNNB1 were identified and combined with three types of 5'UTR (CCNE1, RAC1, and PDCD4) to construct IVT-RNA luciferase reporters (FIG. 6(A)), and the translation efficiencies thereof were then evaluated.

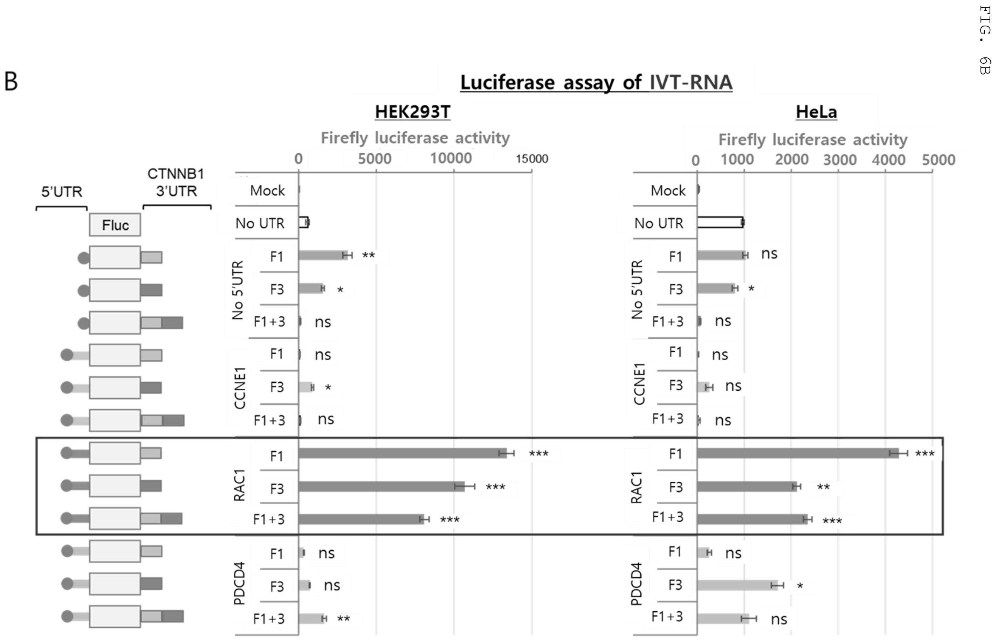

The results are shown in FIG. 6.

As shown in FIG. 6, luciferase reporter assay results confirmed that translation efficiency was superior only when the RAC1 5'UTR and the CTNNB1 3'UTR fragments were combined in both HEK293T and HeLa cell lines.

In other words, the above experiments confirmed that the 5'UTR of RAC1 should be included as a sequence that effectively enhances translation efficiency.

### Example 5. Validation of Optimal Fragments of the RAC1 5'UTR and CTNNB1 3'UTR

The optimal combinations of the RAC1 5'UTR and CTNNB1 3'UTR fragments, which showed the highest expression efficiencies, were validated.

Specifically, the full-length (FL) and SL3 sequences of the RAC1 5'UTR were utilized, along with the F1, F3, and F1+F3 positions of the CTNNB1 3'UTR and the HBB full-length (FL) 3'UTR as a control.

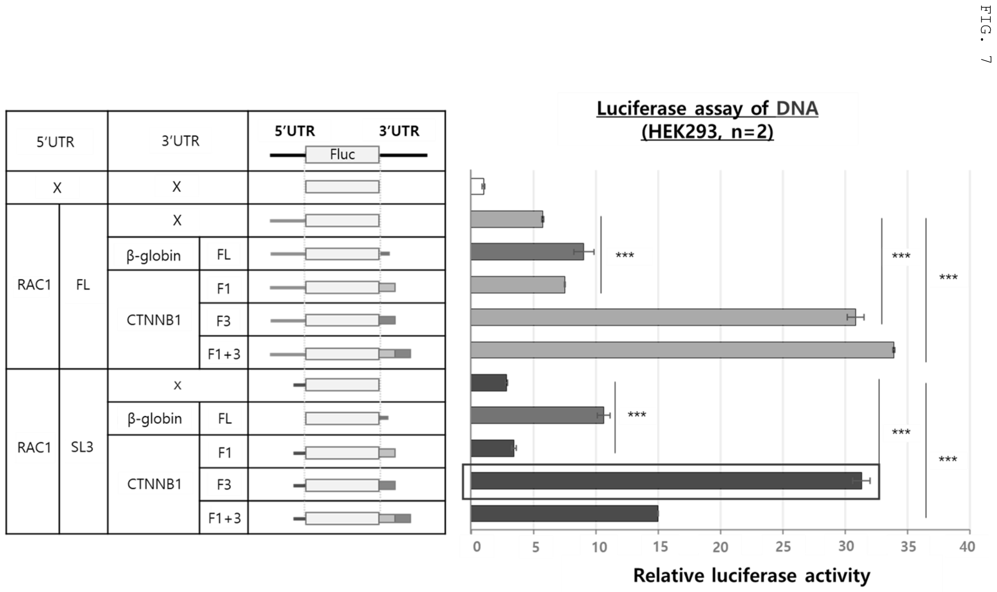

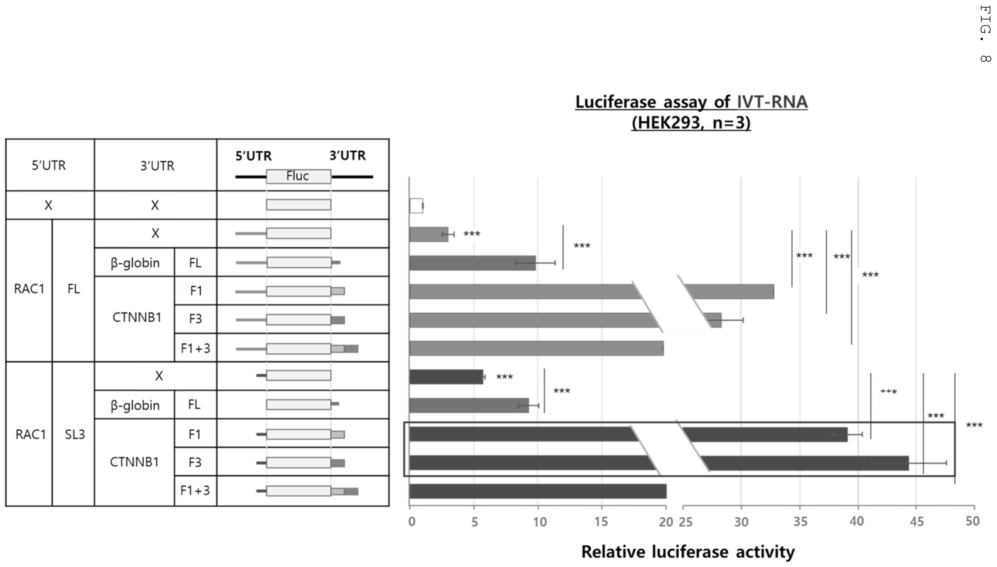

As in the previous method, experiments were conducted using DNA luciferase reporters and IVT-RNA luciferase reporters, and the results are shown in FIGS. 7 and 8.

As shown in FIGS. 7 and 8, both the full-length (FL) and SL3 sequences of the RAC1 5'UTR showed enhanced translation efficiency compared to those without these sequences.

In particular, this enhancement in translation efficiency was observed more significantly in combinations of the SL3 sequence of the RAC1 5'UTR and the F1, F3, and F1+F3 sequences of the CTNNB1 3'UTR. In other words, when the SL3 sequence of the RAC1 5'UTR and the β-catenin 3'UTR or fragments thereof were used together, a synergistic translation enhancement effect was observed.

### Example 6. Translation Efficiency Analysis of G-quadruplex Mutations in RAC1 5'UTR

QGRS Mapper analysis predicted two candidate G-quadruplex (GQ) sequences within the RAC1 5'UTR, designated Gq1 and Gq2, respectively. For functional validation, the Gq2 region, which exhibited a high G-score, was selected as the target sequence.

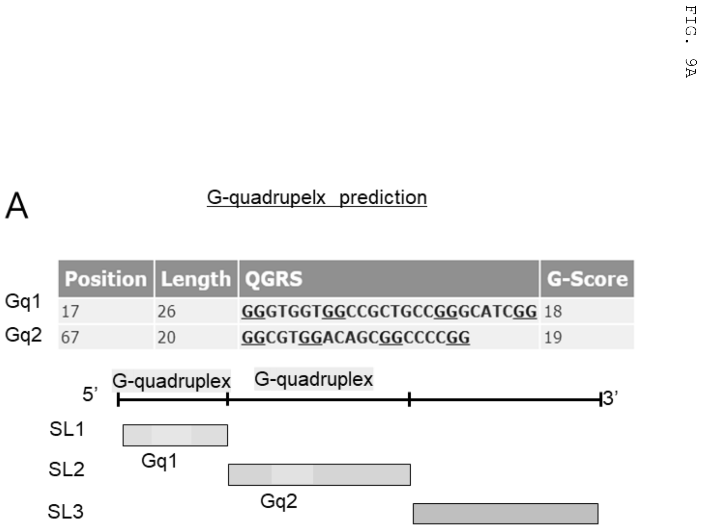

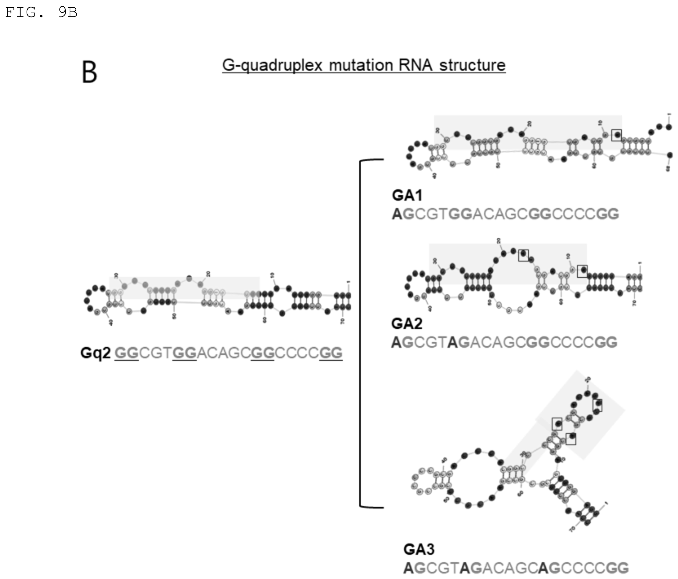

To substitute guanine (G) in the G-rich sequence of Gq2 with adenine (A), site-directed mutagenesis PCR was performed. Using primers encompassing the mutation site, the mutant fragment was amplified in the first PCR, and the entire RAC1 5'UTR was amplified in the second PCR to obtain the final mutant sequence. The obtained mutant sequences were designated GA1, GA2, and GA3 according to the number of mutations (FIG. 9).

The RAC1 5'UTR mutant sequences and their corresponding primer sequences are shown in Tables 9 and 10, respectively. The portion where the guanine (G) sequence was substituted with adenine (A) is indicated in bold.

**[Table 9]**

| **Name** | **Sequence (5'→3'**) |
|---|---|
| GA1 | AGTTTTCCTCAGCTTTGGGTGGTGGCCGCTGCCGGGCATCGGC TTCCAGTCCGCGGAGGGCGAGGCAGCGTGGACAGCGGCCCCGG CACCCAGCGCCCCGCCGCCCGCAAGCCGCGCGCCCGTCCGCCG CGCCCCGAGCCCGCCGCTTCCTATCTCAGCGCCCTGCCGCCGC CGCCGCGGCCCAGCGAGCGGCCCTG (SEQ ID NO: 52) |
| GA2 | AGTTTTCCTCAGCTTTGGGTGGTGGCCGCTGCCGGGCATCGGC TTCCAGTCCGCGGAGGGCGAGGCAGCGTAGACAGCGGCCCCGG CACCCAGCGCCCCGCCGCCCGCAAGCCGCGCGCCCGTCCGCCG CGCCCCGAGCCCGCCGCTTCCTATCTCAGCGCCCTGCCGCCGC CGCCGCGGCCCAGCGAGCGGCCCTG (SEQ ID NO: 53) |
| GA3 | AGTTTTCCTCAGCTTTGGGTGGTGGCCGCTGCCGGGCATCGGC TTCCAGTCCGCGGAGGGCGAGGCAGCGTAGACAGCAGCCCCGG CACCCAGCGCCCCGCCGCCCGCAAGCCGCGCGCCCGTCCGCCG CGCCCCGAGCCCGCCGCTTCCTATCTCAGCGCCCTGCCGCCGC CGCCGCGGCCCAGCGAGCGGCCCTG (SEQ ID NO: 54) |

**[Table 10]**

| **Name** | **Sequence (5'-3')** |
|---|---|
| RAC1_ | **A**GCGTGGACAGCGGCCCCGG (SEQ ID NO: 55) |
| GA1_MUTA Forward | |
| RAC1_ GA1_MUTA Reverse | CCGGGGCCGCTGTCCACGC**T** (SEQ ID NO: 56) |
| RAC1_ GA2_MUTA Forward | **A**GCGT**A**GACAGCGGCCCCGG (SEQ ID NO: 57) |
| RAC1_ GA2_MUTA Reverse | CCGGGGCCGCTGTC**T**ACGC**T** (SEQ ID NO: 58) |
| RAC1_ GA3 MUTA Forward | **A**GCGT**A**GACAGC**A**GCCCCGG (SEQ ID NO: 59) |
| RAC1_ GA3 MUTA Reverse | CCGGGGC**T**GCTGTC**T**ACGC**T** (SEQ ID NO: 60) |

The luciferase reporter vector was constructed based on the pGL3 control vector. All RAC1 5'UTR sequences were amplified by PCR using the RAC1 5'UTR DNA as a template, with primers designed to include HindIII and NcoI restriction enzyme recognition sequences. The amplified products were cloned by ligation into the pGL3 vector, which had been digested with the same restriction enzymes.

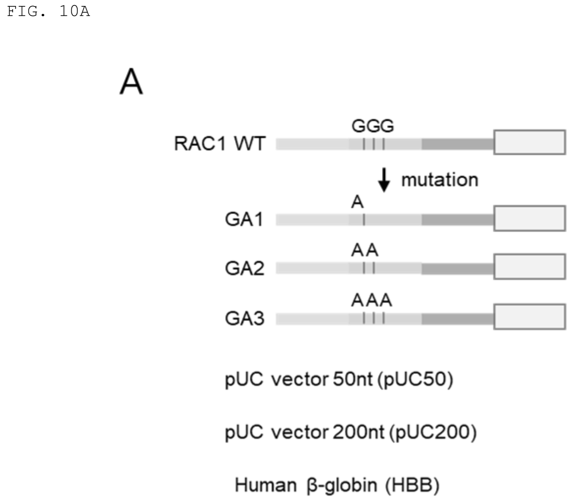

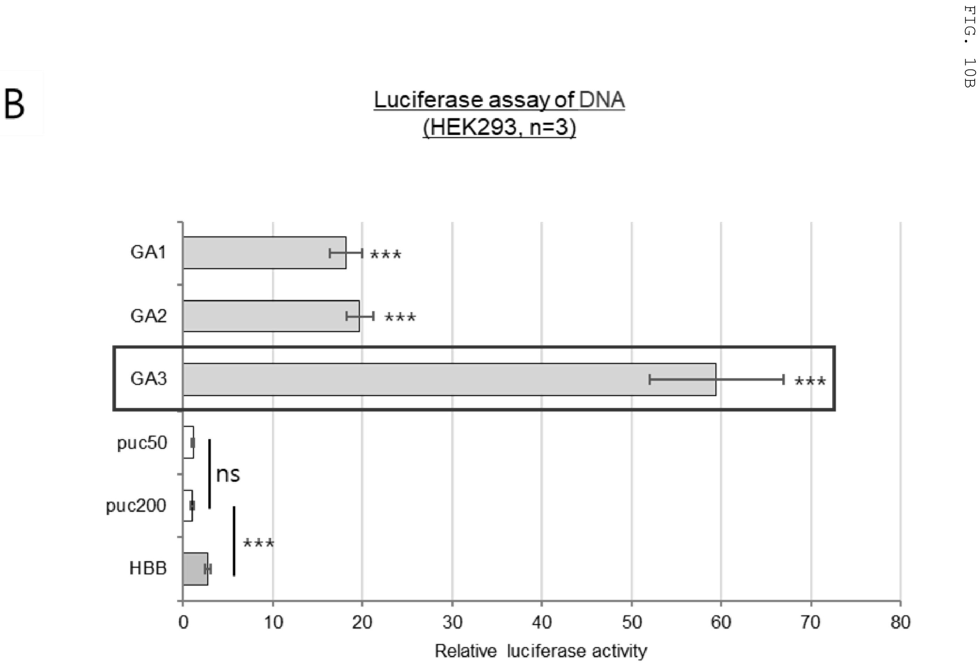

When comparing translation activity by introducing mutant reporters into HEK293 cells, the GA3 mutant with the highest G→A substitution ratio exhibited the strongest translation activity (FIG. 10). These results suggest that the destabilization of the GQ structure may have removed repressive structural elements within the RAC1 5'UTR or induced a structural shift favorable for translation enhancement.

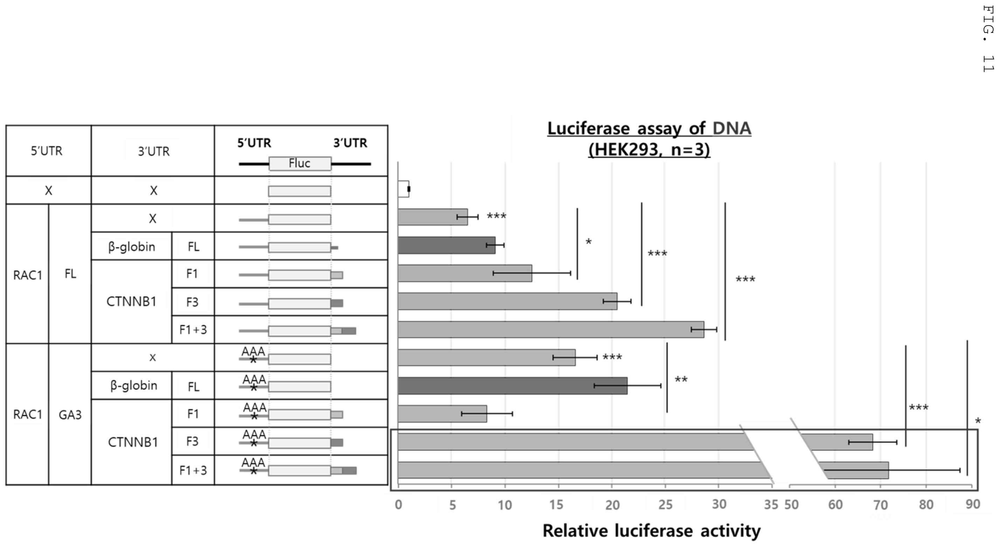

Next, the RAC1 5'UTR mutant sequences were combined with the CTNNB1 3'UTR fragments, and translation activities thereof were analyzed. As a result, the highest translation activity was observed in the GA3(5'UTR)-F3(3'UTR) and GA3(5'UTR)-F1+3(3'UTR) combinations (FIG. 11). This suggests that the translation-enhancing effect of RAC1 5'UTR, into which the GQ mutation was introduced, may be further promoted by combining with the CTNNB1 3'UTR fragment.

In conclusion, the above experiments confirmed that translation efficiency increased when the G-quadruplex-related guanine (G) residues in the RAC1 5'UTR sequence were mutated, and this effect was particularly prominent in the DNA-based expression platform.

## Claims

1. A nucleic acid construct comprising:
(a) a coding region encoding a target protein;
(b) a 5' untranslated region (5'UTR) of Ras-related C3 botulinum toxin substrate 1 (RAC1) or a fragment thereof, linked to the 5' end of the coding region; and
(c) a 3' untranslated region (3'UTR) or a fragment thereof, linked to the 3' end of the coding region.

2. The nucleic acid construct of claim 1, wherein the 5'UTR or a fragment thereof comprises any one sequence selected from the group consisting of SEQ ID NOs: 30 to 36.

3. The nucleic acid construct of claim 1 or claim 2, wherein the 5'UTR of RAC1 is a mutant in which one or more guanine (G) residues in a G-rich sequence forming a G-quadruplex (GQ) structure are substituted with adenine (A), uracil (U), or cytosine (C).

4. The nucleic acid construct according to any one of claims 1 to 3, wherein the mutant comprises any one sequence selected from the group consisting of SEQ ID NOs: 52 to 54.

5. The nucleic acid construct according to any one of claims 1 to 4, wherein the 3'UTR or a fragment thereof is any one 3'UTR or a fragment thereof selected from the group consisting of β-catenin, pUC, β-actin, and human β-globin.

6. The nucleic acid construct according to any one of claims 1 to 5, wherein the 3'UTR or a fragment thereof is a 3'UTR of β-catenin or a fragment thereof, and comprises any one sequence selected from the group consisting of SEQ ID NOs: 23 to 25.

7. The nucleic acid construct of according to any one of claims 1 to 6, wherein the 5'UTR or a fragment thereof comprises any one sequence selected from the group consisting of SEQ ID NOs: 30 to 36, and
the 3'UTR or a fragment thereof comprises any one sequence selected from the group consisting of SEQ ID NOs: 23 to 25.

8. The nucleic acid construct according to any one of claims 1 to 7, wherein the nucleic acid construct comprises a 5' cap structure at the 5' end of the 5'UTR, a poly (A) tail structure at the 3' end of the 3'UTR, or both.

9. The nucleic acid construct according to any one of claims 1 to 8, wherein the nucleic acid construct comprises at least any one modified nucleotide selected from the group consisting of N1-methylpseudo uridine, and 5-methyl cytosine.

10. The nucleic acid construct of according to any one of claims 1 to 9, wherein the nucleic acid construct is RNA or DNA.

11. A recombinant vector comprising the nucleic acid construct of any one of claims 1 to 10.

12. An isolated host cell comprising the recombinant vector of claim 11.

13. A method for producing a nucleic acid through in vitro transcription using the recombinant vector of claim 11 as a template.

14. A composition comprising the nucleic acid construct of any one of claims 1 to 10.

15. A pharmaceutical composition comprising the nucleic acid construct of any one of claims 1 to 10; and a pharmaceutically acceptable carrier.
